# EUROPEAN PATENT APPLICATION

(11) **EP 4 417 627 A1**
(43) Date of publication of application: **21.08.2024**
(21) Application number: 22880163.5
(22) Date of filing: 28.09.2022
(51) Int. Cl.: C07K 19/00, C07K 16/30, A61K 39/395

(54) **RECOMBINANT FUSION ANTIBODY, AND ANTIBODY-DRUG CONJUGATE AND USE THEREOF**

(30) Priority: 15.10.2021 CN 202111201616
(71) Applicant: Sun Yat-Sen University, Guangzhou, Guangdong 510275 (CN)
(72) Inventor: CAI, Xiaoqing, Guangzhou, Guangdong 510006 (CN); JIANG, Xianxing, Guangzhou, Guangdong 510006 (CN)
(74) Representative: Meyer, Thorsten
(86) International application number: PCT/CN2022/122143
(87) International publication number: WO 2023/061224

(57) **Abstract**

Disclosed are a recombinant fusion antibody, and an antibody-drug conjugate and use thereof. The recombinant fusion antibody includes an antibody, at least one lysosome-targeting peptide, and at least one linker peptide, wherein the antibody is fused with the lysosome-targeting peptide to form the recombinant fusion antibody via the linker peptide; wherein the lysosome-targeting peptide is at least one selected from the group consisting of a lysosome-sorting signal peptide from a Golgi-localized, γ-adaptin ear-containing, ARF-binding protein (GGA), a lysosome-sorting signal peptide from a mannose 6-phosphate receptor (MPR), a lysosome-sorting signal peptide from a lysosome-associated membrane protein (LAMP), and a variant; wherein the variant is a peptide with one or more amino acids substituted, deleted, and/or added when aligned with the lysosome-sorting signal peptide from a GGA, a MPR or a LAMP. Compared with the original antibody, the internalization and lysosomal trafficking ability of the recombinant fusion antibody are significantly improved. The recombinant-fusion-antibody-drug conjugate shows an excellent anti-tumor activity in experiments and can be used for cancer treatment.

## Description

### TECHNICAL FIELD

The present disclosure belongs to the technical field of biological medicines, and relates to a recombinant fusion antibody and an antibody-drug conjugate thereof, in particular to a novel recombinant fusion antibody, and an antibody-drug conjugate containing the recombinant fusion antibody and use thereof.

### BACKGROUND ART

An antibody-drug conjugate (ADC), is a macromolecular conjugate that comprises an antibody conjugated to a cytotoxic agent via a small-molecule linker. The conjugate delivers toxic small molecules to tumor cells by the targeting effect of an antibody, thereby reducing the toxicity and side effects caused by the non-specificity of a conventional chemotherapeutic drug. ADCs combine the advantages of chemotherapy and antibody-based targeted therapy, and thus provide a new research approach for precise targeted cancer therapy. In the past two decades, ADCs have developed rapidly and become one of the hotspots in the global research and development of antitumor drugs. ADCs have the characteristics of high efficiency, low toxicity, a broad disease indication, et al., and have a huge potential for application in the market.

How to improve the endocytosis and lysosomal targeting efficiency of the ADC is a key bottleneck for ADC design. Different than small-molecule drugs, the efficacy of ADCs requires the specific binding of the antibody to an antigen on the cell surface, followed by antigen-mediated endocytosis and transportation to lysosomes for ADC degradation and release of the small-molecule toxic drugs, which kill tumor cells. After being presented on the surface of tumor cells, not all ADC molecules can be efficiently endocytosed; and only a portion of the ADCs that are endocytosed into the cells can ultimately be degraded by the lysosomal pathway. Most ADCs enter the cells via three pathways: clathrin-mediated endocytosis, caveolin-mediated endocytosis, and macropinocytosis. Among these three pathways, only clathrin-mediated endocytosis can transport the antibody most efficiently to lysosomes. Therefore, efficient endocytosis and trafficking to lysosomes is essential for the efficacy of ADCs.

The endocytosis and lysosomal trafficking efficiency of the ADC are also limited by the expression level of the antigens on the surface of the tumor cells. Most ADCs in clinics have an average drug-to-antibody ratio of 2 to 4. Generally, at least 10⁴ surface antigens per cell are required to ensure enough amounts of toxic drug molecules delivered by ADCs to cancer cells to reach the minimum efficacious dose. The ideal target antigen for an effective ADC should not only be expressed with sufficient amounts on the surfaces of tumor cells (> 10⁵ antigens/cell), but also be able to induce efficient endocytosis of ADCs. However, the number of antigens on the surfaces of cancer cells is limited and always in a dynamic state of shuttling inside and outside of the cell membrane, therefore a portion of endocytosed ADCs are eventually recycled outside of the cell membrane. Therefore, the number of toxic drugs released inside the cells is very limited. Especially in the cancer cells with low expression levels of antigens, the amount of the small-molecule drug released inside cells is small, therefore ADCs exhibit low cell-killing potency. Taken together, the amount of toxic drugs released from ADCs into cancer cells is often minimal, which is thought to be one of the main reasons for the clinical failure of most ADCs.

How to improve the lysosomal trafficking efficiency of ADCs for the effective release of toxic drugs is one of the bottlenecks in ADC development.

### SUMMARY

In order to solve the problems existing in the prior art, the present disclosure provides a recombinant fusion antibody. The recombinant fusion antibody can improve the internalization efficiency and the lysosomal trafficking efficiency. The present disclosure further provides a recombinant-fusion-antibody-drug conjugate containing the recombinant fusion antibody. The recombinant-fusion-antibody-drug conjugate shows an excellent anti-tumor activity and can be used for cancer treatment. The present disclosure further provides a use of the recombinant-fusion-antibody-drug conjugate in the preparation of a medicament for treating cancer.

The present disclosure is mainly realized by the following technical solutions:
On the one hand, the present disclosure provides a recombinant fusion antibody. The recombinant fusion antibody includes an antibody, at least one lysosome-targeting peptide, and at least one linker peptide, wherein the antibody is fused with the lysosome-targeting peptide to form the recombinant fusion antibody via the linker peptide; wherein the lysosome-targeting peptide is at least one selected from the group consisting of a lysosome-sorting signal peptide from a Golgi-localized, γ-adaptin ear-containing, ARF-binding protein (GGA), a lysosome-sorting signal peptide from a mannose 6-phosphate receptor (MPR), a lysosome-sorting signal peptide from a lysosome-associated membrane protein (LAMP), and a variant; wherein the variant is a peptide with one or more amino acids substituted, deleted, and/or added when aligned with the lysosome-sorting signal peptide from a GGA, a MPR or a LAMP.

The lysosome-targeting peptide of the present disclosure is at least one selected from the group consisting of the following amino acid sequences: ASVSLLDDELMSL (SEQ ID NO: 1), RRRASVSLLDDELMSL (SEQ ID NO: 2), ASVSLLDDEL (SEQ ID NO: 3), ASSGLDDLDLLGK (SEQ ID NO: 4), VQNPSADRNLLDL (SEQ ID NO: 5), NALSWLDEELLCL (SEQ ID NO: 6), SDEDLLHI (SEQ ID NO: 7), SFHDDSDEDLLHI (SEQ ID NO: 8), EESEERDDHLLPM (SEQ ID NO: 9), SYKYSKVNKE (SEQ ID NO: 10), PAAYRGVGDD (SEQ ID NO: 11), RRRSDEDLLHI (SEQ ID NO: 12), RRLRKSDEDLLHI (SEQ ID NO: 13), RRRRKSDEDLLHI (SEQ ID NO: 14), RRRSFHDDSDEDLLHI (SEQ ID NO: 15), RRLRKSFHDDSDEDLLHI (SEQ ID NO: 16), RRRRKSFHDDSDEDLLHI (SEQ ID NO: 17), RKRSHAGYQTI (SEQ ID NO: 18), RRRRKRKRSHAGYQTI (SEQ ID NO: 19), KHHHAGYEQF (SEQ ID NO: 20), and RRLRKHHHAGYEQF (SEQ ID NO: 21).

The amino acid sequence of the linker peptide of the present disclosure is (Leu-Pro-Glu-Thr)ₓ-(Gly_{y1}-Ser_{y2})_{z}, wherein x = 0 or 1, y₁ = 3, 4 or 5, y₂ = 0 or 1, and z = 1, 2 or 3.

Preferably, the linker peptide of the present disclosure is at least one selected from the group consisting of the following amino acid sequences: Leu-Pro-Glu-Thr-Gly-Gly-Gly (SEQ ID NO: 22), Gly-Gly-Gly, Gly-Gly-Gly-Gly-Gly (SEQ ID NO: 23), Gly-Gly-Gly-Gly-Ser (SEQ ID NO: 24), Gly-Gly-Gly-Gly-Ser-Gly-Gly-Gly-Gly-Ser (SEQ ID NO: 25), and Gly-Gly-Gly-Gly-Ser-Gly-Gly-Gly-Gly-Ser-Gly-Gly-Gly-Gly-Ser (SEQ ID NO: 26).

The antibody of the present disclosure has a tumor-antigen specificity. The antibody is capable of binding to a target protein on the surface of a tumor cell. Preferably, the target protein on the surface of the tumor cell is at least one selected from the group consisting of the following: EGFR, EGFRvIII, HER2, ErB3, CD19, CD20, CD30, CD33, CD37, CD46, CD48, CD74, CD79B, CD27 ligand, TROP2, Nectin-4, PSMA, BCMA, HGFR, FOLR1, CA125, ALCAM, SLC1A5, Siglec-2, Siglec-3, IL-2R alpha, IL-3R alpha, TNF-alpha, B7-H3, GUCY2C, TPBG, ROR2, ENPP3, Coagulation Factor III, Mesothelin, Transferrin R, CEACAM-5, IGF-IR, Syndecan-1, DLL3, EpCAM, LIV-1, ROR1, TIM-1, Ly6E, and NCAM-1.

The antibody of the present disclosure is a monospecific antibody, a bispecific antibody, or a multispecific antibody.

The antibody of the present disclosure is a chimeric antibody, a humanized antibody, or a human antibody.

The antibody of the present disclosure includes a monoclonal antibody or an antigen-binding fragment thereof.

Preferably, the antigen-binding fragment is Fab, Fab', F(ab')₂, Fv, dsFv, scFv, sc(Fv)z, or VHH.

A fusion site of the lysosome-targeting peptide of the present disclosure includes at least one selected from the group consisting of a C-terminus of a heavy chain of the monoclonal antibody, an N-terminus of the heavy chain of the monoclonal antibody, a C-terminus of a light chain of the monoclonal antibody, and an N-terminus of the light chain of the monoclonal antibody.

A fusion site of the lysosome-targeting peptide of the present disclosure includes at least one selected from the group consisting of a C-terminus of the antigen-binding fragment and an N-terminus of the antigen-binding fragment.

On the other hand, the present disclosure provides a recombinant-fusion-antibody-drug conjugate, having the structure as follows:
Drₙ₁Ab,
wherein Dr is a drug; Ab is the recombinant fusion antibody; and n1 is an integer greater than or equal to 1.

The drug of the present disclosure is a cytotoxic agent selected from any one of the following: a microtubule inhibitor or a prodrug thereof, a DNA damaging agent or a prodrug thereof, an RNA polymerase II inhibitor, and a toxin from the bacterial, fungal or animal origin.

Preferably, the microtubule inhibitor is selected from any one of the following: auristatin or a derivative and an analogue thereof, maytansinoid or a derivative and an analogue thereof, paclitaxel or a derivative and an analogue thereof, vinca-alkaloid or a derivative and an analogue thereof, cryptophycin or a derivative and an analogue thereof, and tubulysin or a derivative and an analogue thereof.

Preferably, the DNA damaging agent is selected from any one of the following: doxorubicin or a derivative and an analogue thereof, calicheamicin or a derivative and an analogue thereof, camptothecin or a derivative and an analogue thereof, pyrrolobenzodiazepines (PBD) or a derivative and an analogue thereof, and duocarmycin or a derivative and an analogue thereof.

The drug of the present disclosure is conjugated to a reactive group on the recombinant fusion antibody through a covalent bond.

Preferably, the reactive group on the recombinant fusion antibody is a lysine, a cysteine or a bioorthogonal group.

Preferably, the cysteine is a native cysteine from the recombinant fusion antibody or an engineered cysteine obtained by artificial mutation.

Preferably, the bioorthogonal group is one of an azide group, an alkynyl group, an aldehyde group, a ketone group, and a fluorosulfate group.

The recombinant fusion antibody of the present disclosure is conjugated to the drug via a cleavable or non-cleavable small-molecule linker.

Preferably, the small-molecule linker is selected from any one of the following: a peptide, an oligosaccharide, -(CH₂)ₙ-, -(CH₂CH₂O)ₙ-, Val-Cit-PABC, Val-Ala-PABC, Val-Lys(Ac)-PABC, Phe-Lys-PABC, Phe-Lys(Ac)-PABC, D-Val-Leu-Lys, Gly-Gly-Arg, Ala-Ala-Asn-PABC, Ala-PABC, and PABC or a combination thereof, wherein n is an integer of 1-10, Cit is citrulline, Ac is acetyl, and PABC is p-aminobenzyl alcohol.

On the other hand, the present disclosure provides a use of the recombinant-fusion-antibody-drug conjugate in the preparation of a medicament for treating cancer.

The cancer of the present disclosure is a gastric cancer, an intestinal cancer, a liver cancer, a lung cancer, a pancreatic cancer, a breast cancer, a cervical cancer, an endometrial cancer, an ovarian cancer, a prostate cancer, a bladder cancer, a nasopharyngeal/throat or soft tissue cancer, a blood cancer or lymphoma, and a skin cancer.

Compared with the prior art, the technical solution of the present disclosure has the following advantages:

The recombinant fusion antibody of the present disclosure is prepared by fusing a lysosome-targeting peptide at a C-terminus and/or an N-terminus of an antibody. The lysosome-targeting peptide is a human lysosome-sorting signal peptide or a peptide mutant based on a human lysosome-sorting signal peptide. Compared with the original antibody, the recombinant fusion antibody of the present disclosure can enhance the endocytosis efficiency and lysosomal trafficking activity of the recombinant-fusion-antibody-drug conjugate, which can facilitate the effective release of a drug inside the cell, and improve the anti-tumor activity of the recombinant-fusion-antibody-drug conjugate. The recombinant-fusion-antibody-drug conjugate of the present disclosure possesses a high endocytosis efficiency and lysosomal trafficking ability, shows excellent anti-tumor activity in experiments, and can be used for treating cancer.

### BRIEF DESCRIPTION OF THE DRAWINGS

FIG. 1 is a schematic diagram of the structures of the three nanobodies with fusion of a lysosome-targeting peptide described in Example 1 of the present disclosure;
FIG. 2 is a schematic diagram of the structures of six single-chain antibodies with fusion of a lysosome-targeting peptide described in Example 1 of the present disclosure;
FIG. 3 is a schematic diagram of the structures of fifteen full-length antibody with fusion of a lysosome-targeting peptide described in Example 1 of the present disclosure;
FIG. 4 is the SDS-PAGE analysis of the recombinant fusion nanobodies described in Example 1 of the present disclosure and recombinant-fusion-nanobody-drug conjugates described in Example 4 of the present disclosure;
FIG. 5 is the SDS-PAGE analysis of the recombinant fusion full-length antibodies described in Example 1 of the present disclosure and recombinant-fusion-full-length-antibody-drug conjugates described in Example 4 of the present disclosure;
FIG. 6A shows the characterization results of the internalization of the recombinant fusion nanobodies VHH1-VHH6 described in Example 2 of the present disclosure;
FIG. 6B shows the quantification results of the internalization of the recombinant fusion nanobodies VHH1-VHH6 described in Example 2 of the present disclosure;
FIG. 7A shows the characterization results of the internalization of the recombinant fusion nanobodies VHH7-VHH22 described in Example 2 of the present disclosure;
FIG. 7B shows the quantification results of the internalization of the recombinant fusion nanobodies VHH7-VHH22 described in Example 2 of the present disclosure;
FIG. 8A shows the characterization results of the internalization of the recombinant fusion nanobodies VHH23-VHH26 described in Example 2 of the present disclosure;
FIG. 8B shows the quantification results of the internalization of the recombinant fusion nanobodies VHH23-VHH26 described in Example 2 of the present disclosure;
FIG. 9A shows the characterization results of the internalization of the recombinant fusion full-length antibodies described in Example 2 of the present disclosure;
FIG. 9B shows the quantification result of the internalization of the recombinant fusion full-length antibodies described in Example 2 of the present disclosure;
FIG. 10A shows the confocal laser scanning microscope images of the fused VHH1-VHH6 described in Example 3 of the present disclosure;
FIG. 10B shows the Pearson's correlation coefficients between the fused VHH1-VHH6 and lysosomes described in Example 3 of the present disclosure;
FIG. 11A shows the confocal laser scanning microscope images of the fused VHH7-VHH22 described in Example 3 of the present disclosure;
FIG. 11B shows the Pearson's correlation coefficients between the fused VHH7-VHH22 and lysosomes described in Example 3 of the present disclosure;
FIG. 12A shows the confocal laser scanning microscope images of the fused VHH23-VHH26 described in Example 3 of the present disclosure;
FIG. 12B shows the Pearson's correlation coefficients between the fused VHH23-VHH26 and lysosomes described in Example 3 of the present disclosure;
FIG. 13A shows the confocal laser scanning microscope images of the recombinant fusion full-length antibodies described in Example 3 of the present disclosure;
FIG. 13B shows the Pearson's correlation coefficients between the recombinant fusion full-length antibodies and lysosomes described in Example 3 of the present disclosure;
FIG. 14A shows the cell viability of SKBR3 cells treated with the recombinant-fusion-nanobody-drug conjugates, fused VHH1-MMAF to fused VHH6-MMAF, described in Example 5 of the present disclosure;
FIG. 14B shows the cell viability of SKBR3 cells treated with the recombinant-fusion-nanobody-drug conjugates, fused VHH7-MMAF to fused VHH22-MMAF, described in Example 5 of the present disclosure;
FIG. 14C shows the cell viability of SKBR3 cells treated with the recombinant-fusion-nanobody-drug conjugates, fused VHH23-MMAF to fused VHH26-MMAF, described in Example 5 of the present disclosure;
FIG. 15 shows the cell viability of MDA-MB-231 cells treated with the fused VHH7-MMAF and fused VHH8-MMAF as a negative control described in Example 5 of the present disclosure;
FIG. 16 shows the cell viability of SKBR3 cells treated with the recombinant-fusion-full-length-antibody-drug conjugates described in Example 5 of the present disclosure;
FIG. 17 shows the cell viability of SKOV3 cells treated with the recombinant-fusion full-length-antibody-drug conjugates described in Example 5 of the present disclosure;
FIG. 18 shows the images of SKOV3 cells treated with the recombinant-fusion-full-length-antibody-drug conjugates described in Example 5 of the present disclosure;
FIG. 19 shows the in vivo anti-tumor activity of recombinant-fusion-antibody-drug conjugates in the BT474 human breast cancer model described in Example 6 of the present disclosure ;
FIG. 20 shows the in vivo anti-tumor activity of the recombinant-fusion-antibody-drug conjugates in the NCI-N87 human gastric cancer model described in Example 6 of the present disclosure.

### DETAILED DESCRIPTION

Embodiments of the present disclosure will be described in detail below with reference to examples, but those skilled in the art would understand that the following examples are only used to illustrate the present disclosure and should not be regarded as limiting the scope of the present disclosure. If no specific conditions are specified in the examples, the examples will be conducted according to conventional conditions or the conditions recommended by the manufacturer. If not specified with the manufacturer, the used reagents or instruments are all commercially available common products.

### Lysosome-targeting peptide

The lysosome-targeting peptide of the present disclosure is a lysosome-sorting signal peptide from a Golgi-localized, γ-adaptin ear-containing, ARF-binding protein (GGA), a lysosome-sorting signal peptide from a mannose 6-phosphate receptor (MPR), and a lysosome-sorting signal peptide from a lysosome-associated membrane protein (LAMP), or a variant; the variant is a peptide with one or more amino acids substituted, deleted, and/or added when aligned with the lysosome-sorting signal peptide from a GGA, a MPR or a LAMP. The amino acid sequences of the preferred lysosome-targeting peptides of the present disclosure are shown in the following Table 1, but the lysosome-targeting peptides of the present disclosure include, but are not limited to the amino acid sequences shown in Table 1.

| **Table 1 Amino acid sequences of lysosome-targeting peptides** | | | |
|---|---|---|---|
| **Lysosome-targeting peptide** | | **Amino acid sequence** | **SEQ ID NO:** |
| Based on GGA | P1 | ASVSLLDDELMSL | 1 |
| | P2 | RRRASVSLLDDELMSL | 2 |
| | P3 | ASVSLLDDEL | 3 |
| | P4 | ASSGLDDLDLLGK | 4 |
| | P5 | VQNPSADRNLLDL | 5 |
| | P6 | NALSWLDEELLCL | 6 |
| Based on MPR | P7 | SDEDLLHI | 7 |
| | P8 | SFHDDSDEDLLHI | 8 |
| | P9 | EESEERDDHLLPM | 9 |
| | P10 | SYKYSKVNKE | 10 |
| | P11 | PAAYRGVGDD | 11 |
| | P12 | RRRSDEDLLHI | 12 |
| | P13 | RRLRKSDEDLLHI | 13 |
| | P14 | RRRRKSDEDLLHI | 14 |
| | P15 | RRRSFHDDSDEDLLHI | 15 |
| | P16 | RRLRKSFHDDSDEDLLHI | 16 |
| | P17 | RRRRKSFHDDSDEDLLHI | 17 |
| Based on LAMP | P18 | RKRSHAGYQTI | 18 |
| | P19 | RRRRKRKRSHAGYQTI | 19 |
| | P20 | KHHHAGYEQF | 20 |
| | P21 | RRLRKHHHAGYEQF | 21 |

### Linker peptide

The amino acid sequence of the linker peptide of the present disclosure is (Leu-Pro-Glu-Thr)ₓ-(Gly_{y1}-Ser_{y2})_{z}, wherein x = 0 or 1, y₁ = 3, 4 or 5, y₂ = 0 or 1, and z = 1, 2 or 3. The preferred linker peptides of the present disclosure include, but are not limited to the amino acid sequences shown in the following Table 2:

| **Table 2 Amino acid sequences of linker peptides** | | |
|---|---|---|
| **Linker peptide** | **Amino acid sequence** | **SEQ ID NO:** |
| Linker 1 | Leu-Pro-Glu-Thr-Gly-Gly-Gly | 22 |
| Linker2 | Gly-Gly-Gly | - |
| Linker3 | Gly-Gly-Gly-Gly-Gly | 23 |
| Linker4 | Gly-Gly-Gly-Gly-Ser | 24 |
| Linker5 | Gly-Gly-Gly-Gly-Ser-Gly-Gly-Gly-Gly-Ser | 25 |
| Linker6 | Gly-Gly-Gly-Gly-Ser-Gly-Gly-Gly-Gly-Ser-Gly-Gly-Gly-Gly-Ser | 26 |

| | | |
|---|---|---|
| Note: Linker2 has only 3 amino acids. Since the attached sequence listing of amino acids of the specification requires that each polypeptide has at least 4 amino acids, linker peptide Linker2 is not shown in the sequence listing and thus has no corresponding SEQ ID NO:. | | |

### Example 1 Preparation of recombinant fusion antibody

To conveniently describe the recombinant fusion antibody of the present disclosure, an antigen-binding fragment (nanobody, hereinafter abbreviated as VHH) and a monoclonal antibody (full-length antibody, hereinafter abbreviated as mAb) against the anti-human epidermal growth factor receptor 2 (HER2) were selected as model antibodies. The mAb had a heavy chain (HC) mAb-HC and a light chain (LC) mAb-LC. It should be noted that the antibody of the present disclosure included, but was not limited to the above two antibodies.

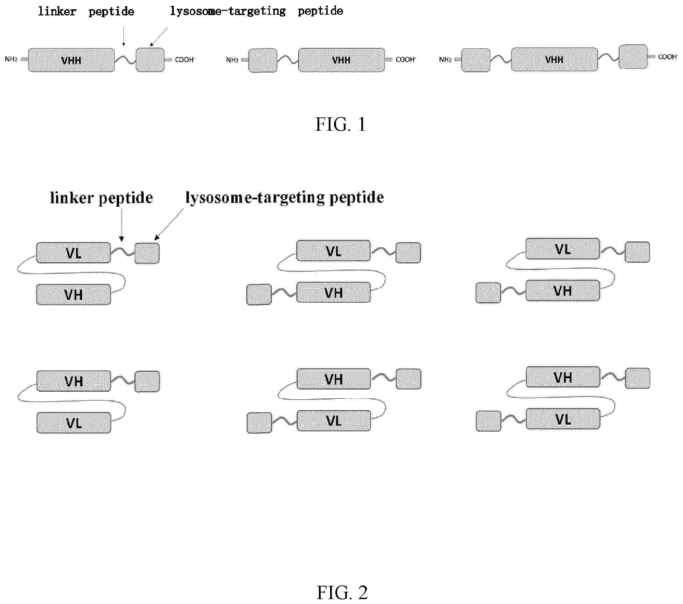

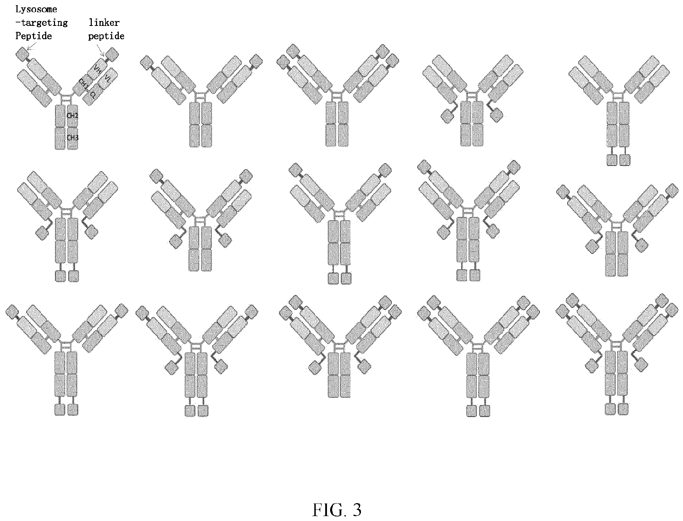

Referring to FIGs. 1-3, FIG. 1 is a schematic diagram of the structures of the three nanobodies with fusion of a lysosome-targeting peptide of the present disclosure; FIG. 2 is a schematic diagram of the structures of the six single-chain antibodies (abbreviated as scFv) with fusion of a lysosome-targeting peptide of the present disclosure; and FIG. 3 is a schematic diagram of the structures of the fifteen full-length antibodies with fusion of a lysosome-targeting peptide of the present disclosure. The fusions of a lysosome-targeting peptide to an antibody of the present disclosure included, but was not limited to the structures shown in FIGs. 1-3. The lysosome-targeting peptide of the present disclosure can be selectively fused to at least one selected from the group consisting of a C-terminus of the antigen-binding fragment of an antibody, an N-terminus of the antigen-binding fragment, a C-terminus of a heavy chain of the monoclonal antibody, an N-terminus of the heavy chain of the monoclonal antibody, a C-terminus of a light chain of the monoclonal antibody, and an N-terminus of the light chain of the monoclonal antibody.

### 1) Site-specific labeling of antibody

To facilitate the linking of a drug to an antibody, the antibody may firstly be site-specifically labeled, for example, by mutating serine to cysteine at position 84 of the VHH to obtain VHH-S84C, and by mutating alanine to cysteine at position 121 of the heavy chain mAb-HC of the mAb to obtain mAb-HC-A121C. The amino acid sequences of VHH, VHH-S84C, mAb-HC, mAb-HC-A121C, and mAb-LC were shown in the following Table 3:

| **Table 3 Amino acid sequences of antibodies** | | |
|---|---|---|
| **Antibody** | **Amino acid sequence** | **SEQ ID NO:** |
| VHH | EVQLVESGGGLVQAGGSLRLSCAASGITFSINTMGWYRQAPG KQRELVALISSIGDTYYADSVKGRFTISRDNAKNTVYLQMNSL KPEDTAVYYCKRFRTAAQGTDYWGQGTQVTVSS | 27 |
| VHH-S84C | EVQLVESGGGLVQAGGSLRLSCAASGITFSINTMGWYRQAPG KQRELVALISSIGDTYYADSVKGRFTISRDNAKNTVYLQMNCL KPEDTAVYYCKRFRTAAQGTDYWGQGTQVTVSS | 28 |
| mAb-HC | EVQLVESGGGLVQPGGSLRLSCAASGFNIKDTYIHWVRQAPG KGLEWVARIYPTNGYTRYADSVKGRFTISADTSKNTAYLQMN SLRAEDTAVYYCSRWGGDGFYAMDYWGQGTLVTVSSASTK GPSVFPLAPSSKSTSGGTAALGCLVKDYFPEPVTVSWNSGALT SGVHTFPAVLQSSGLYSLSSVVTVPSSSLGTQTYICNVNHKPSN TKVDKKVEPKSCDKTHTCPPCPAPELLGGPSVFLFPPKPKDTL MISRTPEVTCVVVDVSHEDPEVKFNWYVDGVEVHNAKTKPR EEQYNSTYRVVSVLTVLHQDWLNGKEYKCKVSNKALPAPIEK TISKAKGQPREPQVYTLPPSREEMTKNQVSLTCLVKGFYPSDI AVEWESNGQPENNYKTTPPVLDSDGSFFLYSKLTVDKSRWQQ GNVFSCSVMHEALHNHYTQKSLSLSPGK | 29 |
| mAb-HC-A121C | EVQLVESGGGLVQPGGSLRLSCAASGFNIKDTYIHWVRQAPG KGLEWVARIYPTNGYTRYADSVKGRFTISADTSKNTAYLQMN SLRAEDTAVYYCSRWGGDGFYAMDYWGQGTLVTVSSCSTK GPSVFPLAPSSKSTSGGTAALGCLVKDYFPEPVTVSWNSGALT SGVHTFPAVLQSSGLYSLSSVVTVPSSSLGTQTYICNVNHKPSN TKVDKKVEPKSCDKTHTCPPCPAPELLGGPSVFLFPPKPKDTL MISRTPEVTCVVVDVSHEDPEVKFNWYVDGVEVHNAKTKPR EEQYNSTYRVVSVLTVLHQDWLNGKEYKCKVSNKALPAPIEK TISKAKGQPREPQVYTLPPSREEMTKNQVSLTCLVKGFYPSDI AVEWESNGQPENNYKTTPPVLDSDGSFFLYSKLTVDKSRWQQ GNVFSCSVMHEALHNHYTQKSLSLSPGK | 30 |
| mAb-LC | DIQMTQSPSSLSASVGDRVTITCRASQDVNTAVAWYQQKPGK APKLLIYSASFLYSGVPSRFSGSRSGTDFTLTISSLQPEDFATYY CQQHYTTPPTFGQGTKVEIKRTVAAPSVFIFPPSDEQLKSGTAS VVCLLNNFYPREAKVQWKVDNALQSGNSQESVTEQDSKDST YSLSSTLTLSKADYEKHKVYACEVTHQGLSSPVTKSFNRGEC | 31 |

### 2) Construction of plasmids for recombinant fusion antibodies

In this example, a lysosome-targeting peptide was fused to the C-terminus of an antibody via a linker peptide using a genetic recombination method.

### a) Construction of the expression plasmid for fused VHH with fusion of a lysosome-targeting peptide

Purification tags such as c-Myc, histidine, GST, MBP, and a lipid tag can be introduced into the C-terminal of the VHH, such that the expressed product is easier to be detected and purified. His₆ was selected as a protein purification tag in this example. The His₆ tag was fused to the C-terminus of VHH-S84C, which was not fused with a lysosome-targeting peptide, to obtain VHH-S84C-His₆, denoted as VHHO; Linker1-Linker4 and Linker6 were selected as linker peptides and P1-P21 as lysosome-targeting peptides, and the purification tag His₆, the linker peptides, and the lysosome-targeting peptides were fused to the C terminal of the site-specific mutant antibody to prepare the recombinant fusion antibodies: fused VHH1-VHH26, whose amino acid sequences are shown in Table 4. But the recombinant fusion antibodies of the present disclosure are not limited to the amino acid sequences given in this example. The linker peptides Linker1-Linker6 and the lysosome-targeting peptides P1-P21 of the present disclosure can be combined at will, and the obtained recombinant fusion antibodies are all within the protection scope of the present disclosure.

The construction of the plasmids for the lysosome-targeting-peptide-fused VHHs of this example specifically included the following steps:

Firstly, the His₆ tag was fused to the C-terminal of VHH-S84C to construct a fragment VHH-S84C-His₆. Then, a linker peptide and a lysosome-targeting peptide were fused, respectively, before or after the His₆ sequence to obtain target fragments of the recombinant fusion antibodies. Finally, the target fragments of the recombinant fusion antibodies were integrated into a vector pET-28a to obtain the expression plasmid for a recombinant fusion antibody.

| **Table 4 Amino acid sequences of recombinant fusion antibodies** | | | |
|---|---|---|---|
| **Target fragment** | **Name** | **Amino acid sequence** | **SEQ ID NO:** |
| VHH-S84C-His₆ | VHH0 | EVQLVESGGGLVQAGGSLRLSCAASGITFSINT MGWYRQAPGKQRELVALISSIGDTYYADSVK GRFTISRDNAKNTVYLQMNCLKPEDTAVYYC KRFRTAAQGTDYWGQGTQVTVSSHHHHHH | 32 |
| VHH-S84C-His₆-Linker1-P1 | Fused VHH1 | EVQLVESGGGLVQAGGSLRLSCAASGITFSINT MGWYRQAPGKQRELVALISSIGDTYYADSVK | 33 |
| | | GRFTISRDNAKNTVYLQMNCLKPEDTAVYYC KRFRTAAQGTDYWGQGTQVTVSSHHHHHHLP ETGGGASVSLLDDELMSL | |
| VHH-S84C-His₆-Linker1-P2 | Fused VHH2 | EVQLVESGGGLVQAGGSLRLSCAASGITFSINT MGWYRQAPGKQRELVALISSIGDTYYADSVK GRFTISRDNAKNTVYLQMNCLKPEDTAVYYC KRFRTAAQGTDYWGQGTQVTVSSHHHHHHLP ETGGGRRRASVSLLDDELMSL | 34 |
| VHH-S84C-His₆-Linker1-P3 | Fused VHH3 | EVQLVESGGGLVQAGGSLRLSCAASGITFSINT MGWYRQAPGKQRELVALISSIGDTYYADSVK GRFTISRDNAKNTVYLQMNCLKPEDTAVYYC KRFRTAAQGTDYWGQGTQVTVSSHHHHHHLP ETGGGASVSLLDDEL | 35 |
| VHH-S84C-His₆-Linker1-P4 | Fused VHH4 | EVQLVESGGGLVQAGGSLRLSCAASGITFSINT MGWYRQAPGKQRELVALISSIGDTYYADSVK GRFTISRDNAKNTVYLQMNCLKPEDTAVYYC KRFRTAAQGTDYWGQGTQVTVSSHHHHHH LPETGGGASSGLDDLDLLGK | 36 |
| VHH-S84C-His₆-Linker1-P5 | Fused VHH5 | EVQLVESGGGLVQAGGSLRLSCAASGITFSINT MGWYRQAPGKQRELVALISSIGDTYYADSVK GRFTISRDNAKNTVYLQMNCLKPEDTAVYYC KRFRTAAQGTDYWGQGTQVTVSSHHHHHHLP ETGGGVQNPSADRNLLDL | 37 |
| VHH-S84C-His₆-Linker1-P6 | Fused VHH6 | EVQLVESGGGLVQAGGSLRLSCAASGITFSINT MGWYRQAPGKQRELVALISSIGDTYYADSVK GRFTISRDNAKNTVYLQMNCLKPEDTAVYYC KRFRTAAQGTDYWGQGTQVTVSSHHHHHH LPETGGGNALSWLDEELLCL | 38 |
| VHH-S84C-His₆-Linker1-P7 | Fused VHH7 | EVQLVESGGGLVQAGGSLRLSCAASGITFSINT MGWYRQAPGKQRELVALISSIGDTYYADSVK GRFTISRDNAKNTVYLQMNCLKPEDTAVYYC KRFRTAAQGTDYWGQGTQVTVSSHHHHHHLP ETGGGSDEDLLHI | 39 |
| VHH-S84CLinker1-P7-His₆ | Fused VHH8 | EVQLVESGGGLVQAGGSLRLSCAASGITFSINT MGWYRQAPGKQRELVALISSIGDTYYADSVK GRFTISRDNAKNTVYLQMNCLKPEDTAVYYC KRFRTAAQGTDYWGQGTQVTVSSLPETGGGS DEDLLHIHHHHHH | 40 |
| VHH-S84C-His₆-Linker2-P7 | Fused VHH9 | EVQLVESGGGLVQAGGSLRLSCAASGITFSINT MGWYRQAPGKQRELVALISSIGDTYYADSVK GRFTISRDNAKNTVYLQMNCLKPEDTAVYYC KRFRTAAQGTDYWGQGTQVTVSSHHHHHHG GGSDEDLLHI | 41 |
| VHH-S84C-His₆-Linker3-P7 | Fused VHH10 | EVQLVESGGGLVQAGGSLRLSCAASGITFSINT MGWYRQAPGKQRELVALISSIGDTYYADSVK | 42 |
| | | GRFTISRDNAKNTVYLQMNCLKPEDTAVYYC KRFRTAAQGTDYWGQGTQVTVSSHHHHHHG GGGGSDEDLLHI | |
| VHH-S84C-His₆-Linker4-P7 | Fused VHH11 | EVQLVESGGGLVQAGGSLRLSCAASGITFSINT MGWYRQAPGKQRELVALISSIGDTYYADSVK GRFTISRDNAKNTVYLQMNCLKPEDTAVYYC KRFRTAAQGTDYWGQGTQVTVSSHHHHHHG GGGSSDEDLLHI | 43 |
| VHH-S84C-His₆-Linker6-P7 | Fused VHH12 | EVQLVESGGGLVQAGGSLRLSCAASGITFSINT MGWYRQAPGKQRELVALISSIGDTYYADSVK GRFTISRDNAKNTVYLQMNCLKPEDTAVYYC KRFRTAAQGTDYWGQGTQVTVSSHHHHHHG GGGSGGGGSGGGGSSDEDLLHI | 44 |
| VHH-S84C-His₆-Linker1-P8 | Fused VHH13 | EVQLVESGGGLVQAGGSLRLSCAASGITFSINT MGWYRQAPGKQRELVALISSIGDTYYADSVK GRFTISRDNAKNTVYLQMNCLKPEDTAVYYC KRFRTAAQGTDYWGQGTQVTVSSHHHHHHLP ETGGGSFHDDSDEDLLHI | 45 |
| VHH-S84C-His₆-Linker1-P9 | Fused VHH 14 | EVQLVESGGGLVQAGGSLRLSCAASGITFSINT MGWYRQAPGKQRELVALISSIGDTYYADSVK GRFTISRDNAKNTVYLQMNCLKPEDTAVYYC KRFRTAAQGTDYWGQGTQVTVSSHHHHHHLP ETGGGEESEERDDHLLPM | 46 |
| VHH-S84C-His₆-Linker1-P10 | Fused VHH15 | EVQLVESGGGLVQAGGSLRLSCAASGITFSINT MGWYRQAPGKQRELVALISSIGDTYYADSVK GRFTISRDNAKNTVYLQMNCLKPEDTAVYYC KRFRTAAQGTDYWGQGTQVTVSSHHHHHHLP ETGGGSYKYSKVNKE | 47 |
| VHH-S84C-His₆-Linker1-P11 | Fused VHH16 | EVQLVESGGGLVQAGGSLRLSCAASGITFSINT MGWYRQAPGKQRELVALISSIGDTYYADSVK GRFTISRDNAKNTVYLQMNCLKPEDTAVYYC KRFRTAAQGTDYWGQGTQVTVSSHHHHHHLP ETGGGPAAYRGVGDD | 48 |
| VHH-S84C-His₆-Linker1-P12 | Fused VHH17 | EVQLVESGGGLVQAGGSLRLSCAASGITFSINT MGWYRQAPGKQRELVALISSIGDTYYADSVK GRFTISRDNAKNTVYLQMNCLKPEDTAVYYC KRFRTAAQGTDYWGQGTQVTVSSHHHHHHLP ETGGGRRRSDEDLLHI | 49 |
| VHH-S84C-His₆-Linker1-P13 | Fused VHH18 | EVQLVESGGGLVQAGGSLRLSCAASGITFSINT MGWYRQAPGKQRELVALISSIGDTYYADSVK GRFTISRDNAKNTVYLQMNCLKPEDTAVYYC KRFRTAAQGTDYWGQGTQVTVSSHHHHHHLP ETGGGRRLRKSDEDLLHI | 50 |
| VHH-S84C-His₆-Linker1-P14 | Fused VHH 19 | EVQLVESGGGLVQAGGSLRLSCAASGITFSINT MGWYRQAPGKQRELVALISSIGDTYYADSVK GRFTISRDNAKNTVYLQMNCLKPEDTAVYYC KRFRTAAQGTDYWGQGTQVTVSSHHHHHHLP ETGGGRRRRKSDEDLLHI | 51 |
| VHH-S84C-His₆-Linker1-P15 | Fused VHH20 | EVQLVESGGGLVQAGGSLRLSCAASGITFSINT MGWYRQAPGKQRELVALISSIGDTYYADSVK GRFTISRDNAKNTVYLQMNCLKPEDTAVYYC KRFRTAAQGTDYWGQGTQVTVSSHHHHHHLP ETGGGRRRSFHDDSDEDLLHI | 52 |
| VHH-S84C-His₆-Linker1-P16 | Fused VHH21 | EVQLVESGGGLVQAGGSLRLSCAASGITFSINT MGWYRQAPGKQRELVALISSIGDTYYADSVK GRFTISRDNAKNTVYLQMNCLKPEDTAVYYC KRFRTAAQGTDYWGQGTQVTVSSHHHHHHLP ETGGGRRLRKSFHDDSDEDLLHI | 53 |
| VHH-S84C-His₆-Linker1-P17 | Fused VHH22 | EVQLVESGGGLVQAGGSLRLSCAASGITFSINT MGWYRQAPGKQRELVALISSIGDTYYADSVK GRFTISRDNAKNTVYLQMNCLKPEDTAVYYC KRFRTAAQGTDYWGQGTQVTVSSHHHHHHLP ETGGGRRRRKSFHDDSDEDLLHI | 54 |
| VHH-S84C-His₆-Linker1-P18 | Fused VHH23 | EVQLVESGGGLVQAGGSLRLSCAASGITFSINT MGWYRQAPGKQRELVALISSIGDTYYADSVK GRFTISRDNAKNTVYLQMNCLKPEDTAVYYC KRFRTAAQGTDYWGQGTQVTVSSHHHHHH LPETGGGRKRSHAGYQTI | 55 |
| VHH-S84C-His₆-Linker1-P19 | Fused VHH24 | EVQLVESGGGLVQAGGSLRLSCAASGITFSINT MGWYRQAPGKQRELVALISSIGDTYYADSVK GRFTISRDNAKNTVYLQMNCLKPEDTAVYYC KRFRTAAQGTDYWGQGTQVTVSSHHHHHH LPETGGGRRRRKRKRSHAGYQTI | 56 |
| VHH-S84C-His₆-Linker1-P20 | Fused VHH25 | EVQLVESGGGLVQAGGSLRLSCAASGITFSINT MGWYRQAPGKQRELVALISSIGDTYYADSVK GRFTISRDNAKNTVYLQMNCLKPEDTAVYYC KRFRTAAQGTDYWGQGTQVTVSSHHHHHH LPETGGGKHHHAGYEQF | 57 |
| VHH-S84C-His₆-Linker1-P21 | Fused VHH26 | EVQLVESGGGLVQAGGSLRLSCAASGITFSINT MGWYRQAPGKQRELVALISSIGDTYYADSVK GRFTISRDNAKNTVYLQMNCLKPEDTAVYYC KRFRTAAQGTDYWGQGTQVTVSSHHHHHH LPETGGGRRLRKHHHAGYEQF | 58 |

The target fragments and the vector were amplified respectively through PCR. The primers for the target fragments and the vectors contain the recognition sites for the restriction enzymes, NcoI and BstYI. The PCR products and the plasmid vectors were subjected respectively to restriction digestion at the recognition sites designed on the primers. The PCR products and the plasmid vectors after digestion were ligated in the presence of a ligase, and the products were transfected into competent cells DH5α. The cells were then plated and cultured in an incubator overnight. Single plaques were picked and grown on the next day and finally sequenced to verify the genetic recombination.

### b) Construction of expression plasmids for fused mAbs with fusion of a lysosome-targeting peptide

A lysosome-targeting peptide was fused to the mAb at the site which is at least one selected from the group consisting of a C-terminus of the mAb heavy chain, an N-terminus of the mAb heavy chain, a C-terminus of the mAb light chain, and an N-terminus of the mAb light chain. For ease of exposition, in this example, the non-fused mAb was denoted as mAbO, the light chain of mAbO as mAb-LC, and the heavy chain of mAbO as mAb-HC-A121C; Linker1 was used as a linker peptide, P7 was used as a lysosome-targeting peptide, and Linker1 and P7 were fused to the C-terminal of the light chain and/or heavy chain of the site-specific mutant antibody to prepare the recombinant fusion antibodies: fused mAb1-mAb3, whose amino acid sequences are shown in Table 6. But the recombinant fusion antibodies of the present disclosure are not limited to the amino acid sequences given in this example. The linker peptides Linker1-Linker6 and the lysosome-targeting peptides P1-P21 of the present disclosure can be combined at will, the lysosome-targeting peptides can also be fused to the N-terminal of the heavy chain and/or the light chain, and the obtained recombinant fusion antibodies are all within the protection scope of the present disclosure.

The construction of the plasmids for the lysosome-targeting-peptide-fused mAbs of this example specifically included the following steps:
Linker1 and P7 were fused to the C-terminus of the heavy chain and the C-terminus of the light chain to construct fragments mAb-LC-Linker1-P7 and mAb-HC-A121C-Linker1-P7, whose amino acid sequences are shown in Table 5, and the mAb-LC-Linker1-P7 and the mAb-HC-A121C-Linker1-P7 were respectively integrated into a vector pcDNA 3.4.

| **Table 5 Amino acid sequences of recombinant fusion antibodies** | | |
|---|---|---|
| **Target fragment** | **Amino acid sequence** | **SEQ ID NO:** |
| mAb-LC-Linker1-P7 | DIQMTQSPSSLSASVGDRVTITCRASQDVNTAVAWYQQKPG KAPKLLIYSASFLYSGVPSRFSGSRSGTDFTLTISSLQPEDFAT YYCQQHYTTPPTFGQGTKVEIKRTVAAPSVFIFPPSDEQLKSG TASVVCLLNNFYPREAKVQWKVDNALQSGNSQESVTEQDS KDSTYSLSSTLTLSKADYEKHKVYACEVTHQGLSSPVTKSFN RGECLPETGGGSDEDLLHI | 59 |
| mAb-HC-A121CLinker1-P7 | EVQLVESGGGLVQPGGSLRLSCAASGFNIKDTYIHWVRQAP GKGLEWVARIYPTNGYTRYADSVKGRFTISADTSKNTAYLQ MNSLRAEDTAVYYCSRWGGDGFYAMDYWGQGTLVTVSSC STKGPSVFPLAPSSKSTSGGTAALGCLVKDYFPEPVTVSWNS GALTSGVHTFPAVLQSSGLYSLSSVVTVPSSSLGTQTYICNVN HKPSNTKVDKKVEPKSCDKTHTCPPCPAPELLGGPSVFLFPP KPKDTLMISRTPEVTCVVVDVSHEDPEVKFNWYVDGVEVH NAKTKPREEQYNSTYRVVSVLTVLHQDWLNGKEYKCKVSN KALPAPIEKTISKAKGQPREPQVYTLPPSREEMTKNQVSLTCL VKGFYPSDIAVEWESNGQPENNYKTTPPVLDSDGSFFLYSKL TVDKSRWQQGNVFSCSVMHEALHNHYTQKSLSLSPGKLPET GGGSDEDLLHI | 60 |

The expression plasmids for the mAb light chain (mAb-LC) and heavy chain (mAb-HC-A121C) were used to express the full-length antibody mAb0; the expression plasmids for the lysosome-targeting-peptide-fused mAb light chain (mAb-LC-Linker1-P7) and heavy chain (mAb-HC-A121C) were used to express fused mAb1; the expression plasmids for the mAb light chain (mAb-LC) and fused heavy chain (mAb-HC-A121C-Linker1-P7) were used to express fused mAb2; and the expression plasmids for the fused mAb light chain (mAb-LC-Linker1-P7) and fused heavy chain (mAb-HC-A121C-Linker1-P7) were used to express fused mAb3. The amino acid sequences of the fused mAb1-mAb3 are shown in Table 6.

| **Table 6 Amino acid sequences of recombinant fusion antibodies** | | | |
|---|---|---|---|
| **Name** | **Fragment** | **Amino acid sequence** | **SEQ ID NO:** |
| Fused mAb1 | Light chain: mAb-LC-Linker1-P7 | DIQMTQSPSSLSASVGDRVTITCRASQDVNTAVAWYQQ KPGKAPKLLIYSASFLYSGVPSRFSGSRSGTDFTLTISSLQ PEDFATYYCQQHYTTPPTFGQGTKVEIKRTVAAPSVFIFP PSDEQLKSGTASVVCLLNNFYPREAKVQWKVDNALQSG NSQESVTEQDSKDSTYSLSSTLTLSKADYEKHKVYACEV THQGLSSPVTKSFNRGECLPETGGGSDEDLLHI | 61 |
| | Heavy chain: mAb-HC-A121C | EVQLVESGGGLVQPGGSLRLSCAASGFNIKDTYIHWVRQ APGKGLEWVARIYPTNGYTRYADSVKGRFTISADTSKNT AYLQMNSLRAEDTAVYYCSRWGGDGFYAMDYWGQGT LVTVSSCSTKGPSVFPLAPSSKSTSGGTAALGCLVKDYFP EPVTVSWNSGALTSGVHTFPAVLQSSGLYSLSSVVTVPS SSLGTQTYICNVNHKPSNTKVDKKVEPKSCDKTHTCPPC PAPELLGGPSVFLFPPKPKDTLMISRTPEVTCVVVDVSHE DPEVKFNWYVDGVEVHNAKTKPREEQYNSTYRVVSVL TVLHQDWLNGKEYKCKVSNKALPAPIEKTISKAKGQPR EPQVYTLPPSREEMTKNQVSLTCLVKGFYPSDIAVEWES NGQPENNYKTTPPVLDSDGSFFLYSKLTVDKSRWQQGN VFSCSVMHEALHNHYTQKSLSLSPGK | 62 |
| Fused mAb2 | Light chain: mAb-LC | DIQMTQSPSSLSASVGDRVTITCRASQDVNTAVAWYQQ KPGKAPKLLIYSASFLYSGVPSRFSGSRSGTDFTLTISSLQ PEDFATYYCQQHYTTPPTFGQGTKVEIKRTVAAPSVFIFP PSDEQLKSGTASVVCLLNNFYPREAKVQWKVDNALQSG NSQESVTEQDSKDSTYSLSSTLTLSKADYEKHKVYACEV THQGLSSPVTKSFNRGEC | 63 |
| | Heavy chain: mAb-HC-A121CLinker1-P7 | EVQLVESGGGLVQPGGSLRLSCAASGFNIKDTYIHWVRQ APGKGLEWVARIYPTNGYTRYADSVKGRFTISADTSKNT AYLQMNSLRAEDTAVYYCSRWGGDGFYAMDYWGQGT LVTVSSCSTKGPSVFPLAPSSKSTSGGTAALGCLVKDYFP EPVTVSWNSGALTSGVHTFPAVLQSSGLYSLSSVVTVPS SSLGTQTYICNVNHKPSNTKVDKKVEPKSCDKTHTCPPC PAPELLGGPSVFLFPPKPKDTLMISRTPEVTCVVVDVSHE DPEVKFNWYVDGVEVHNAKTKPREEQYNSTYRVVSVL TVLHQDWLNGKEYKCKVSNKALPAPIEKTISKAKGQPR EPQVYTLPPSREEMTKNQVSLTCLVKGFYPSDIAVEWES NGQPENNYKTTPPVLDSDGSFFLYSKLTVDKSRWQQGN VFSCSVMHEALHNHYTQKSLSLSPGKLPETGGGSDEDLL HI | 64 |
| Fused mAb3 | Light chain: mAb-LC-Linker1-P7 | DIQMTQSPSSLSASVGDRVTITCRASQDVNTAVAWYQQ KPGKAPKLLIYSASFLYSGVPSRFSGSRSGTDFTLTISSLQ PEDFATYYCQQHYTTPPTFGQGTKVEIKRTVAAPSVFIFP PSDEQLKSGTASVVCLLNNFYPREAKVQWKVDNALQSG NSQESVTEQDSKDSTYSLSSTLTLSKADYEKHKVYACEV THQGLSSPVTKSFNRGECLPETGGGSDEDLLHI | 65 |
| | Heavy chain: mAb-HC-A121CLinker1-P7 | EVQLVESGGGLVQPGGSLRLSCAASGFNIKDTYIHWVRQ APGKGLEWVARIYPTNGYTRYADSVKGRFTISADTSKNT AYLQMNSLRAEDTAVYYCSRWGGDGFYAMDYWGQGT LVTVSSCSTKGPSVFPLAPSSKSTSGGTAALGCLVKDYFP EPVTVSWNSGALTSGVHTFPAVLQSSGLYSLSSVVTVPS SSLGTQTYICNVNHKPSNTKVDKKVEPKSCDKTHTCPPC PAPELLGGPSVFLFPPKPKDTLMISRTPEVTCVVVDVSHE DPEVKFNWYVDGVEVHNAKTKPREEQYNSTYRVVSVL TVLHQDWLNGKEYKCKVSNKALPAPIEKTISKAKGQPR EPQVYTLPPSREEMTKNQVSLTCLVKGFYPSDIAVEWES NGQPENNYKTTPPVLDSDGSFFLYSKLTVDKSRWQQGN VFSCSVMHEALHNHYTQKSLSLSPGKLPETGGGSDEDLL HI | 66 |

The target fragments and the vector were amplified respectively through PCR. The primers of the target fragments and the vector contain the recognition sites for the restriction enzymes, NcoI and BstYI. The PCR products and the plasmid vectors were subjected respectively to restriction digestion at the recognition sites designed on the primers. Then, the PCR products and the plasmid vectors after digestion were ligated in the presence of a ligase, and the products were transfected into competent cells DH5α. The cells were then plated and cultured in an incubator overnight. Single plaques were picked and grown on the next day and finally sequenced to verify the genetic recombination.

### 3) Expression and purification of recombinant fusion antibody

In this example, a prokaryotic expression system was selected for the antibody expression of VHH0 and fused VHH1-VHH26; and a eukaryotic expression system was selected for the antibody expression of mAbO and fused mAb1-mAb3.

### a) Expression and purification of VHH0 and fused VHH1-VHH26 in prokaryotic expression system

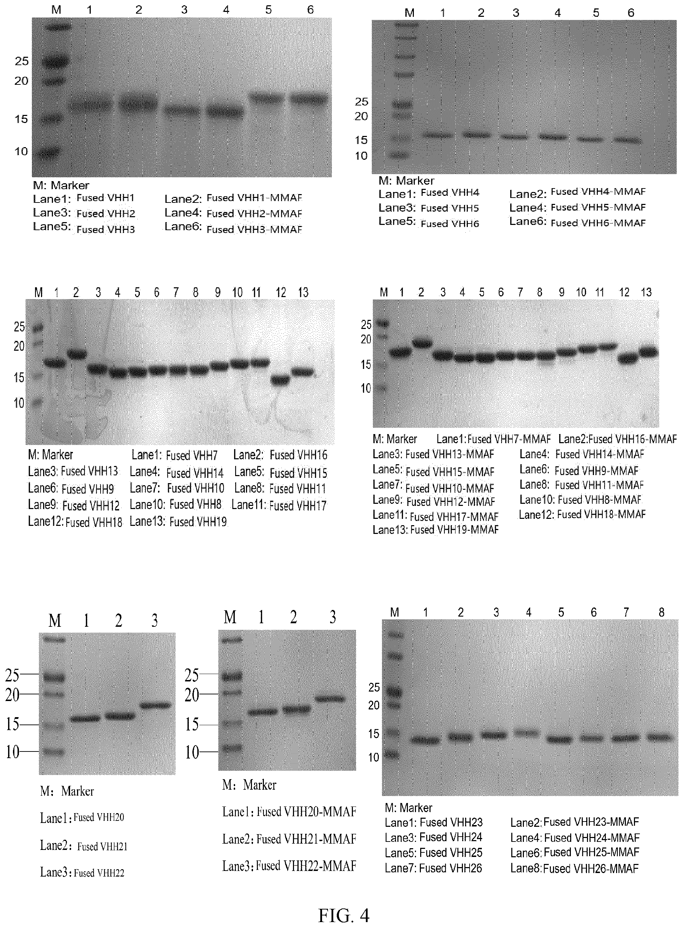

After the expression plasmids for the recombinant fusion antibodies were constructed, the plasmids were transfected into *E.coli* BL21 (DE3) host cells to express the fusion proteins, and the fusion proteins were purified using Ni-NTA beads. Briefly, the Ni-NTA beads were equilibrated with an equilibration buffer (400 mM NaCl, 50 mM Tris-HCl pH 8.0, and 20 mM imidazole), then a sample (cell lysate supernatant) was loaded. Non-specifically bound proteins were washed away with the equilibration buffer, and the protein of interest was eluted with an elution buffer containing 200 mM imidazole and concentrated. Then, the buffer was exchanged, and the protein concentration was determined by a BCA method. The yield of the mutated protein was not significantly changed compared to that of the original protein. The purified proteins were characterized using liquid chromatography-mass spectrometry (LC-MS), matrix-assisted laser desorption/ionization time-of-flight mass spectrometry (MALDI-TOF MS), SDS-PAGE (results are shown in FIG. 4), and HPLC. These results confirm the expression of VHH0 and fused VHH1-VHH26 using the prokaryotic expression system.

### b) Expression and purification of mAbO and fused mAb1-mAb3 in eukaryotic expression system

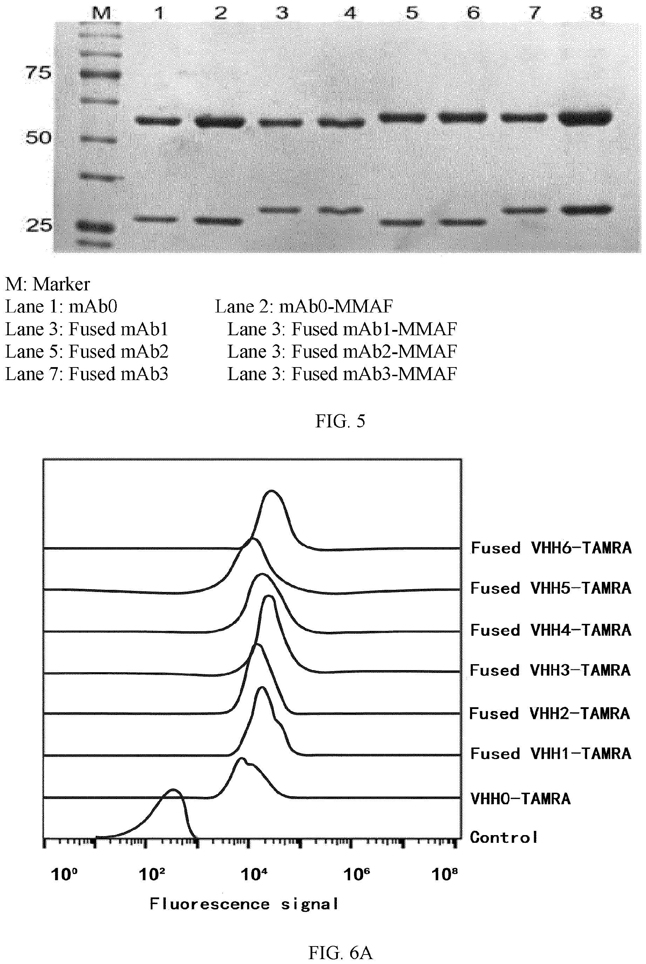

Human embryonic kidney 293 (HEK293) cells were seeded in a shake flask; when the cell density reached 2×10⁶/mL, the cells were transfected using polyethyleneimine (PEI) as a transfection reagent. During transfection, two centrifuge tubes were taken and added with 2 mL phosphate buffered saline (PBS) solution, respectively. Then, the first centrifuge tube was added with 100 µL PEI, the second centrifuge tube was added with 80 µg of the plasmid (heavy chain:light chain = 2:3). Upon complete mixing, a diluent in the second centrifuge tube was added into a PEI diluent in the first centrifuge tube. The transfection reagent complex was vortexed, and incubated for 20 min, and then added to the cells in the shake flask. Cells were incubated for 5 days, and then the supernatant was collected, filtered through a 0.45-µm filter membrane, and purified by a protein A affinity column. The purified proteins were characterized using LC-MS, SDS-PAGE (results are shown in FIG. 5) and HPLC. These results confirm the expression of the mAbO and fused mAb1-mAb3 using the eukaryotic expression system.

### Example 2 Internalization study of recombinant fusion antibody

For the internalization study of the recombinant fusion antibodies, the recombinant fusion antibodies were labeled with a fluorescent probe. The fluorescent molecule includes but is not limited to one of rhodamine, cyanine 3 (CY3), and Texas Red.

In this example, tetramethylrhodamine (TAMRA) was chosen as the fluorescent probe, and was subjected to a Michael Addition reaction with an engineered cysteine residue in the recombinant fusion antibodies via a triethylene glycol-maleimide (PEG3-maleimide) linker.

### 1) Cell culture

BT474, SKBR3, SKOV3, and MDA-MB-231 cell lines (source: American Type Culture Collection (ATCC)) were cultured in a Dulbecco's modified eagle medium (DMEM) (Gibco, USA) supplemented with 10% fetal bovine serum plus penicillin (100 U/mL)-streptomycin (100 g/mL). All the cells were cultured at 37°C under a humidified atmosphere of 5% COz. Cells were morphologically normal and in good growth condition.

### 2) Flow cytometry analysis of the internalization of recombinant fusion antibody

The recombinant fusion antibody was labeled a fluorescent molecule and the internalization activity of the labeled antibody was evaluated by flow cytometry.

In this example, TAMRA with a strong fluorescent signal was used to label VHH0 and fused VHH1-VHH26. The procedure included the following steps: First, 5 mM tris(2-carboxyethyl)phosphine (TCEP; Thermo Fisher Scientific) was added to reduce the sulfhydryl group of VHH0 and fused VHH1-VHH26 respectively for approximately 30 min at room temperature, then TCEP was removed by buffer-exchanging with PBS containing 1 mM ethylenediaminetetraacetic acid (EDTA). Next, 2 eq of Maleimide-PEG3-TAMRA was added to the antibody solution and the resulting solution was incubated at 4°C for 2 h. Lastly, the excess small molecules were removed by buffer-exchanging to obtain TAMRA-labeled VHH0 and fused VHH1-VHH26.

In this example, TAMRA with a strong fluorescent signal was used to label mAbO and fused mAb1-mAb3. The procedure included the following steps: First, dithiothreitol (DTT) was added to the antibody solution and the resulting mixture was incubated at 20°C for 16 h to reduce the sulfhydryl groups on the antibody. The solution was then buffer-exchanged with Tris-buffered saline (50 mM Tris-HCl pH 7.5; 150 mM NaCl) to remove excess DTT. Then, 100 mM dehydroascorbic acid (DHAA) (> 15 eq) was added into the protein solution and the resulting mixture was incubated at room temperature for 3 h. In the reduction-reoxidation step, the natural interchain disulfide bonds were left intact (i.e., reoxidized) and only the engineered cysteines were reduced (i.e., unpaired). Finally, 4 eq of Maleimide-PEG3-TAMRA was added for ligation at 4°C for 2 h, and the excess small molecules were removed by buffer-exchanging to obtain TAMRA-labeled mAbO and fused mAb1-mAb3.

In this example, SKBR3, a human breast cancer cell line with a high expression level of HER2, was used as a model system. TAMRA-labeled antibodies, including VHHO, fused VHH1-VHH26, mAbO, and fused mAb1-mAb3, were diluted with a serum-free medium to a final concentration of 2 µM. SKBR3 cells were incubated with TAMRA-labeled antibodies for 6 h, and subsequently washed with acid to remove bound antibodies on the cell membrane, and the fluorescence intensity was analyzed by flow cytometry.

### a) Internalization efficiency of fused VHH1-VHH26

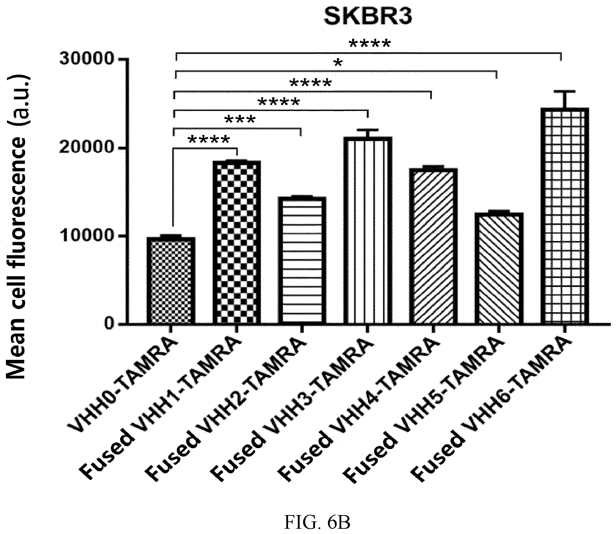

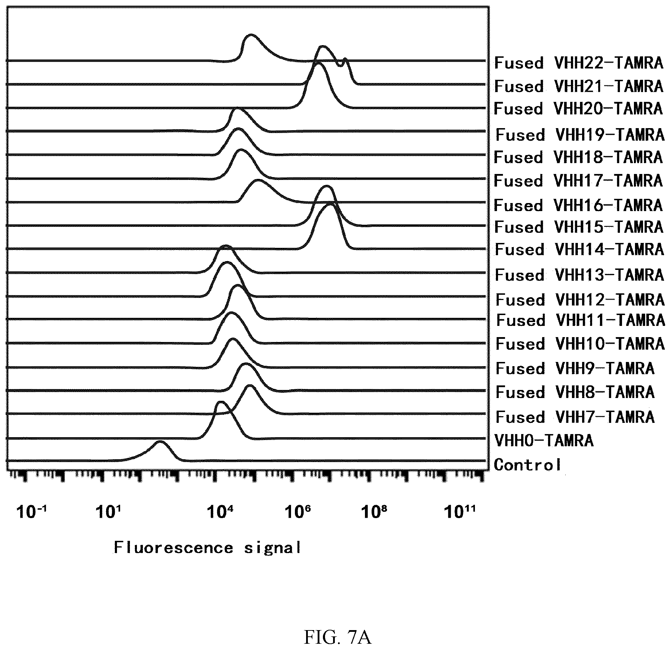

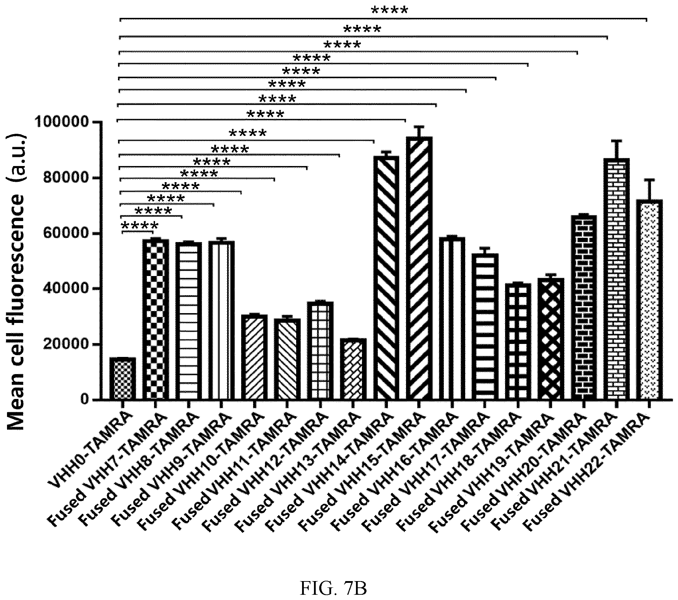

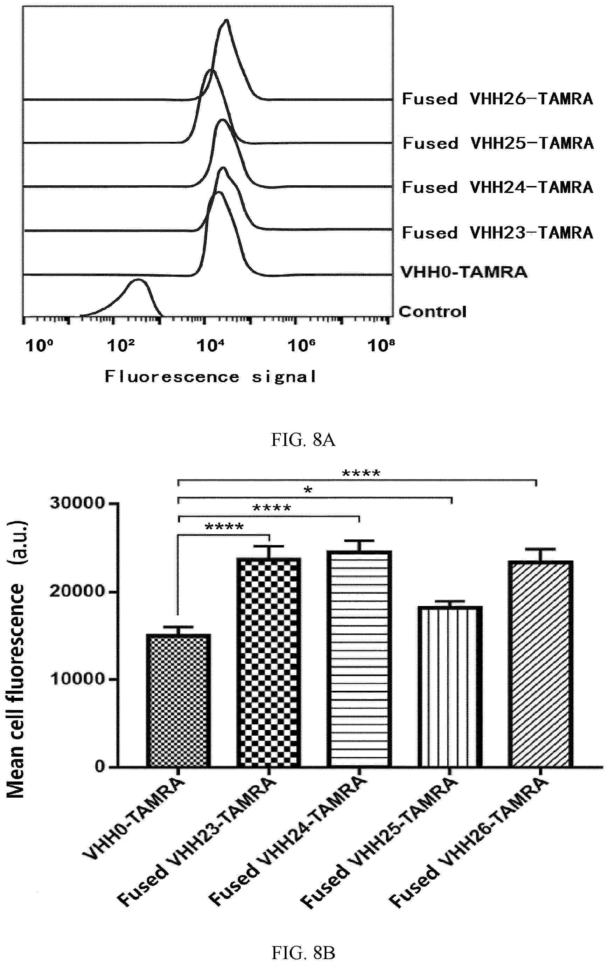

FIGs. 6A, 7A and 8A show the characterization results of the internalization of the recombinant fusion nanobodies VHH1-VHH26, wherein the background fluorescence of untreated cells was use as a control. FIGs. 6B, 7B and 8B show the quantification results of the internalization of the recombinant fusion nanobodies VHH1-VHH26. Data in FIGs. 6B, 7B and 8B were expressed as mean±SEM (n = 3), ns: no significance, **P* < 0.05; ***P* < 0.01; ****P* < 0.001; and *****P* < 0.0001. The flow cytometry analysis and analysis of the mean cell fluorescence values show that lysosome-targeting-peptide-fused VHH1-VHH26 possess significantly higher internalization ability than the non-fused VHH0. These results indicate that the lysosome-targeting peptides can enhance the internalization ability of antibodies. In addition, there was no significant difference in the internalization ability between fused VHH7 and fused VHH8, indicating that the fusion position of the His₆ tag does not affect the internalization ability of the lysosome-targeting peptides.

Furthermore, by comparing the internalization of fused VHH1-VHH7 and fused VHH13-VHH26 with VHHO, it can be seen that fused VHH1-VHH7 and fused VHH13-VHH26 exhibit a higher internalization ability than the non-fused VHH0. Taken together, these results demonstrate that the recombinant fusion of the lysosome-targeting peptides described by the present disclosure can increase the internalization ability of antibodies.

Finally, by comparing the internalization of fused VHH7 and fused VHH9-VHH12, it can be seen that fused VHH7 and fused VHH9-VHH12 exhibit a stronger internalization ability than the non-fused VHH0. Taken together, these results demonstrate that all the linker peptides described by the present disclosure can be used for constructing the recombinant fusion antibodies.

### b) Internalization efficiency of fused mAb1-mAb3

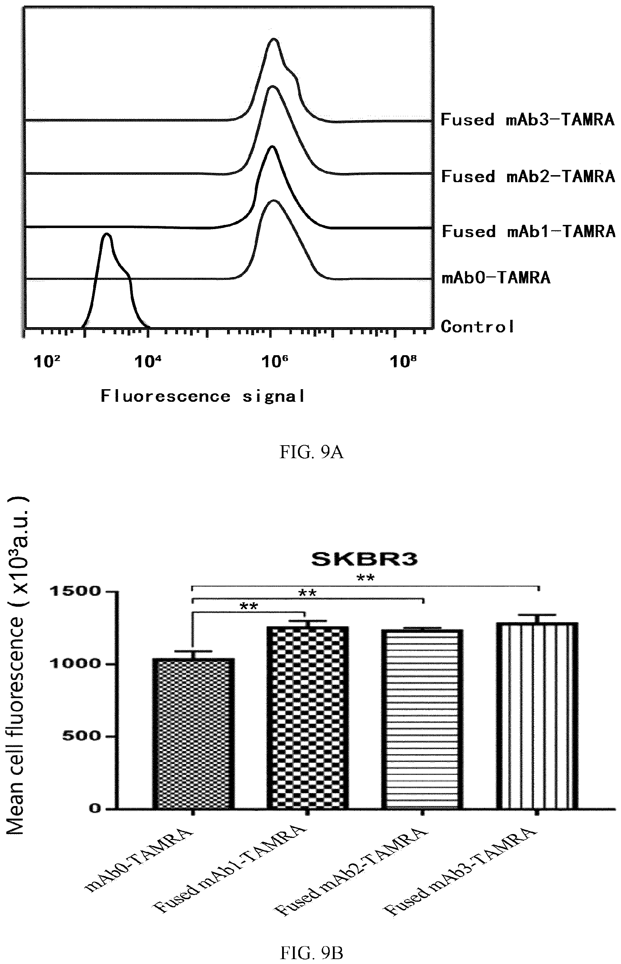

FIG. 9A shows the characterization results of the internalization of the recombinant fusion full-length antibodies, wherein the background fluorescence of untreated cells was used as a control. FIG. 9B shows the quantification results of the internalization of the recombinant fusion full-length antibodies. Data in FIG. 9B were expressed as mean±SEM (n = 3), ns: no significance, ***P* < 0.01; ****P* < 0.001; and *****P* < 0.0001. The flow cytometry analysis and analysis of the mean cell fluorescence values clearly showed that lysosome-targeting-peptide-fused mAb1-mAb3 possess substantially higher internalization ability than the non-fused mAbO.

In addition, fused mAb3, in which the lysosome-targeting peptide P7 is fused to both the light and heavy chains of the mAb, showed the highest internalization efficiency. Fused mAb 1 and Fused mAb2, in which P7 is fused to either light chain or heavy chain of the mAb, also displayed an significantly enhanced internalization ability. No significant difference in the internalization was observed between fused mAb1 and fused mAb2. Therefore, it can be proved that the internalization ability of the antibodies can significantly be enhanced by fusing the antibodies with the lysosome-targeting peptides.

### Example 3 Study of the colocalization of recombinant fusion antibodies with lysosomes

Confocal laser scanning microscope (CLSM) was used to analyze the colocalization of the recombinant fusion antibodies with the lysosome. The antibody-drug conjugate would be transported and processed in the endocytotic pathway as follows: once the antibody-drug conjugate binds to the cell-surface tumor antigen, it is internalized into endosomes, which subsequently mature and fuse with lysosomes. In the lysosomes, the acidic environment and specific proteases (e.g., cathepsin B) degrade the ADC, thus leading to the release of the drug. The free drug traverses the lysosomal membrane, reaches the cytosol and binds to its target, such as microtubule or DNA, thus ultimately triggering cell death. Therefore, the efficacy of an antibody-drug conjugate relies on its internalization and lysosomal trafficking.

In this example, SKBR3 cells were treated with TAMRA-labeled antibodies, including VHHO, fused VHH1-VHH26, mAbO, or fused mAb1-mAb3, for 4 h. The nuclei of SKBR cells were stained by Hoechst 33342 and the lysosomes of cells were stained by LysoTracker Green. The lysosome-trafficking activity of VHHO, fused VHH1-VHH26, mAbO, and fused mAb1-mAb3 was evaluated by confocal laser scanning microscopy. All images were presented under identical conditions. For quantitative analysis, the same threshold value was set for all images.

### 1) Colocalization of fused VHH1-VHH26 with lysosomes

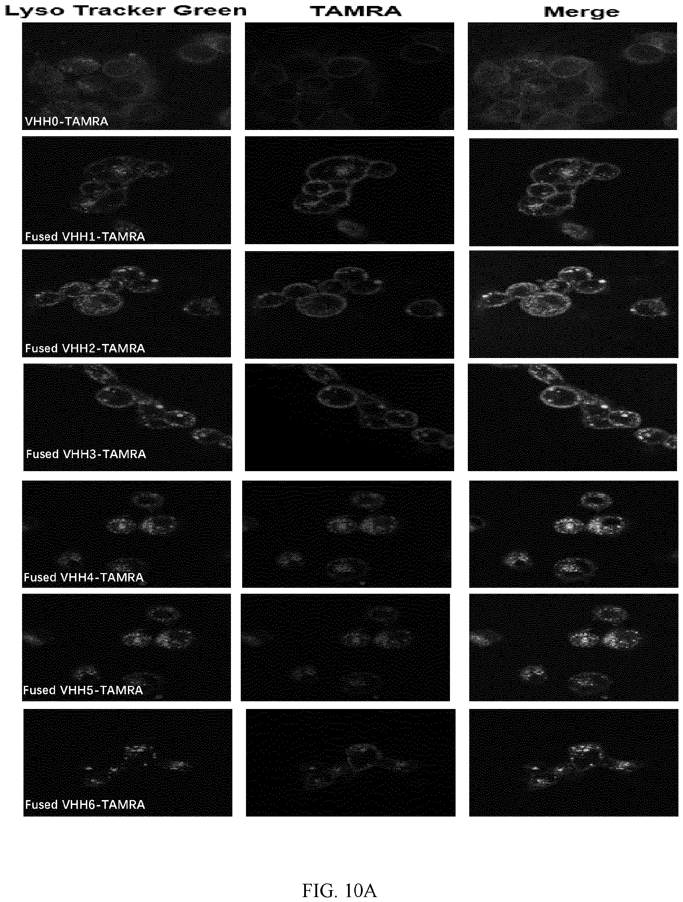

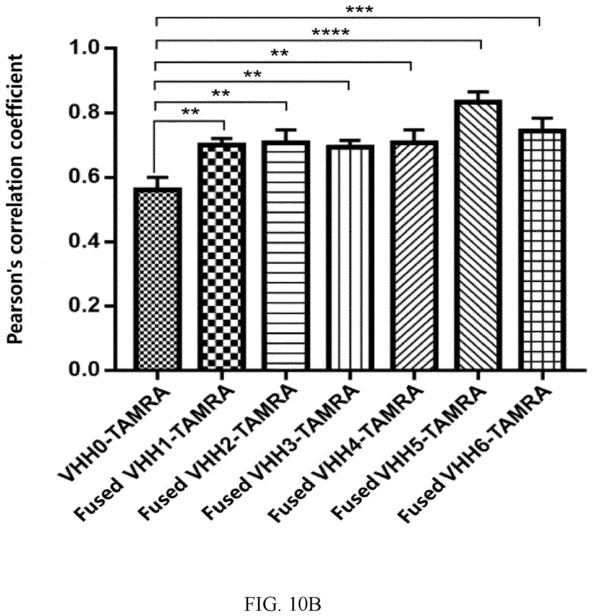

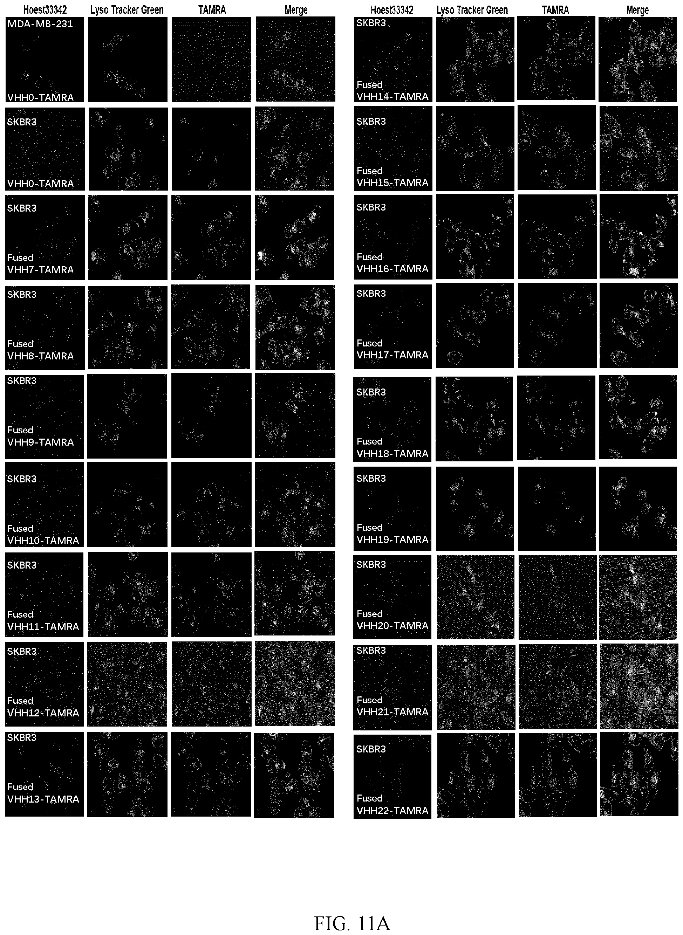

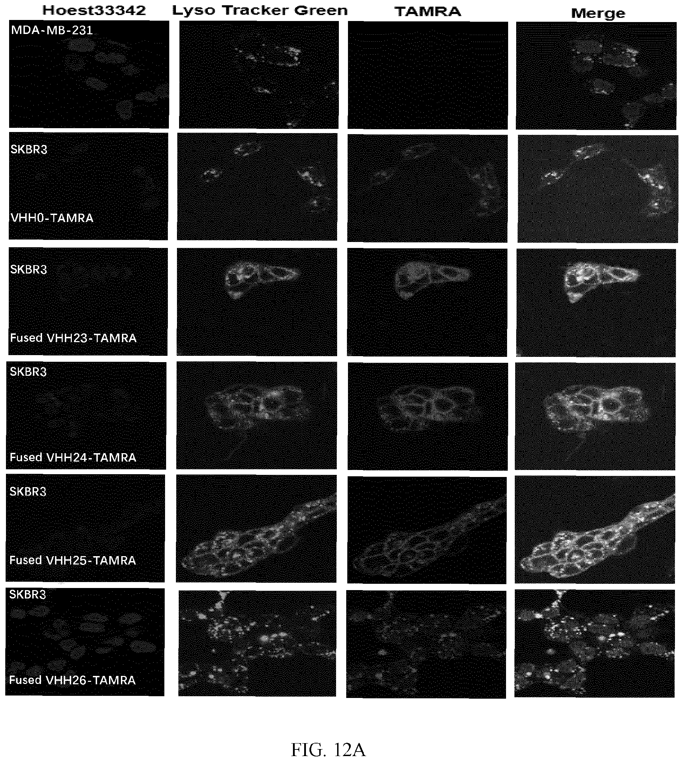

FIG. 10A shows confocal laser scanning microscope images of fused VHH1-VHH6, wherein panels from left to right show lysosomes, antibodies, and the merged images of lysosomes and antibodies, respectively. FIG. 11A shows confocal laser scanning microscope images of fused VHH7-VHH22, wherein panels from left to right show cell nuclei, lysosomes, antibodies, and the merged images of lysosomes and antibodies, respectively. FIG. 12A shows confocal laser scanning microscope images of fused VHH23-VHH26, wherein panels from left to right show cell nuclei, lysosomes, antibodies, and the merged images of lysosomes and antibodies, respectively. FIGs. 10B, 11B and 12B show the Pearson's correlation coefficients between VHH1-VHH26 and lysosomes. Data are shown as mean±SEM; ns: no significance; and **P* < 0.05; ***P* < 0.01; ****P* < 0.001; and *****P* < 0.0001.

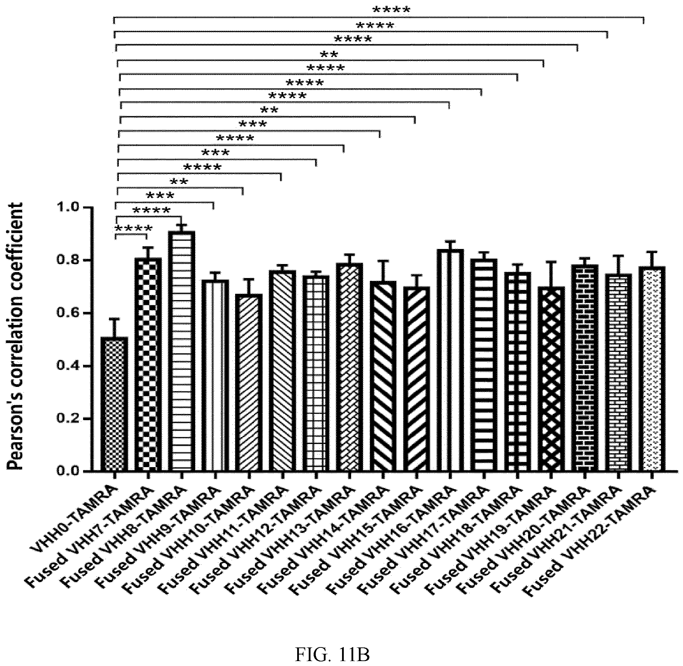

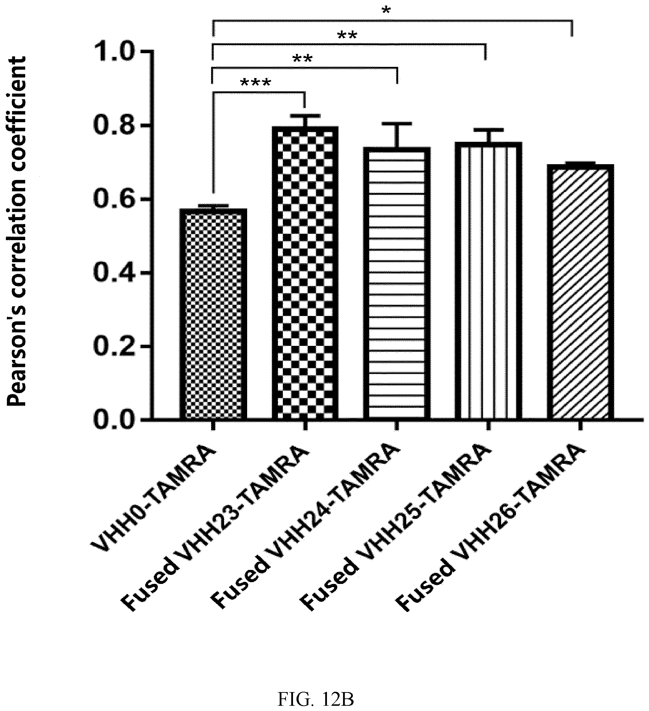

From FIGs. 10A, 11A and 12A, the lysosome-targeting-peptide-fused VHH1-VHH26 show a higher level of colocalization with lysosomes compared to the non-fused VHHO, indicating that fusion of the lysosome-targeting peptides to the antibodies can increase the lysosomal trafficking ability of antibodies. No significant difference in lysosomal trafficking are observed between fused VHH7 and fused VHH8, indicating that the fusion position of the His₆ tag does not affect the lysosome-targeting ability of the lysosome-targeting peptides. From FIGs. 10B, 11B and 12B, the Pearson's correlation coefficients between the recombinant fusion antibodies and lysosomes are consistent with the above results, confirming that the lysosome-targeting-peptide-fused antibodies possess enhanced lysosomal trafficking ability. In addition, FIGs. 11A, 11B, 12A and 12B indicate that the recombinant fusion antibodies show target-specificity against HER2. In contrast, no significant binding was observed for the recombinant fusion antibodies on cell membrane of MDA-MB-231 cells with low HER2 expression.

### 2) Colocalization of fused mAb1-mAb3 with lysosomes

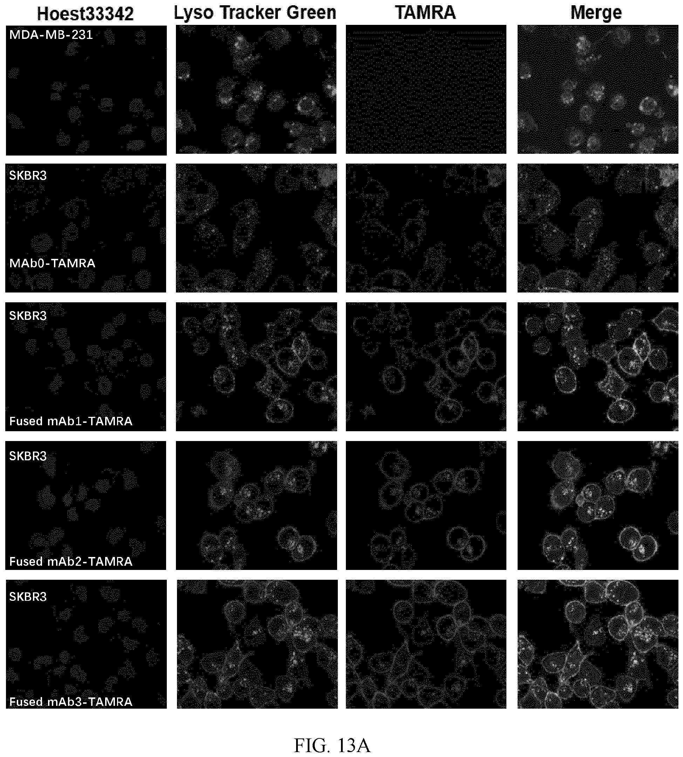

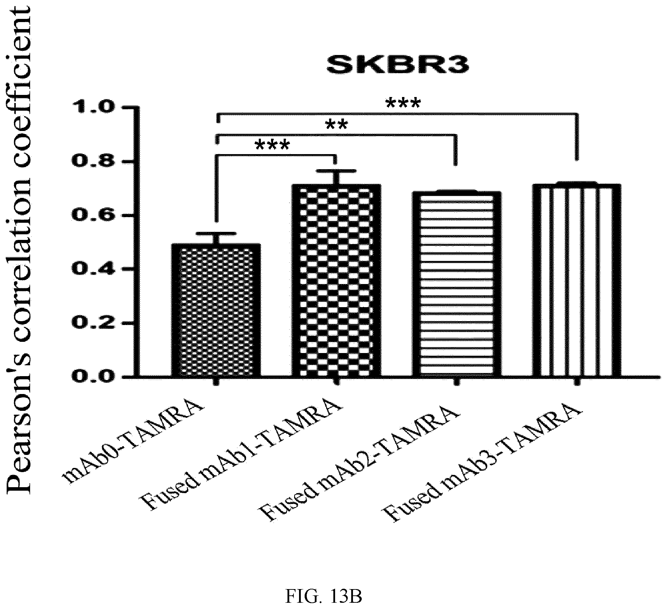

FIG. 13A shows confocal laser scanning microscope images of the recombinant fusion full-length antibodies, wherein panels from left to right show cell nuclei, lysosomes, antibodies, and the merged images of lysosomes and antibodies. FIG. 13B showed the Pearson's correlation coefficients between the recombinant fusion full-length antibodies with lysosomes. Data are expressed as mean±SEM; ns: no significance; ***P* < 0.01; ****P* < 0.001; and *****P* < 0.0001.

From FIG. 13A, the lysosome-targeting-peptide-fused mAb1-mAb3 display higher colocalization with lysosome than the non-fused mAbO, indicating that fusing the lysosome-targeting peptides to the antibodies can significantly increase the ability of the antibodies to target the lysosomes. FIG. 13B shows that the Pearson's correlation coefficients between the recombinant fusion antibodies and lysosomes are also consistent with the above result, confirming that the lysosome-targeting-peptide-fused antibodies possess enhanced lysosomal trafficking ability. Similarly, the results also indicated that the recombinant fusion antibodies show target-specificity against HER2. No significant binding was observed for the recombinant fusion antibodies on the cell membrane of MDA-MB-231 cells with low HER2 expression.

### Example 4 Preparation of recombinant-fusion-antibody-drug conjugates

A drug is conjugated to the recombinant fusion antibody via a small-molecule linker to form the recombinant-fusion-antibody-drug conjugate. The drug is conjugated to a reactive group on the recombinant fusion antibody to form a covalent bond. The reactive group can be a lysine, a cysteine, or a bioorthogonal group. The cysteine can be the native cysteine residues from the recombinant fusion antibody or the cysteine residues engineered onto the antibody. The bioorthogonal group can be an azide group, an alkynyl group, an aldehyde group, a ketone group, or a fluorosulfonate group. The drug is conjugated to the recombinant fusion antibody via a cleavable or non-cleavable linker. The linker is selected from any of the following: a peptide, an oligosaccharide, -(CH₂)ₙ-, -(CH₂CH₂O)ₙ-, Val-Cit-PABC, Val-Ala-PABC, Val-Lys(Ac)-PABC, Phe-Lys-PABC, Phe-Lys(Ac)-PABC, D-Val-Leu-Lys, Gly-Gly-Arg, Ala-Ala-Asn-PABC, Ala-PABC, and PABC or a combination thereof, wherein n is an integer of 1-10, Cit is citrulline, Ac is acetyl, and PABC is p-aminobenzyl alcohol.

The drug of the present disclosure is a cytotoxic agent and includes, but is not limited to any of the following: a microtubule inhibitor or a prodrug thereof, a DNA damaging agent or a prodrug thereof, an RNA polymerase II inhibitor, or a toxin from the bacterial, fungal or animal origin. The microtubule inhibitor includes, but is not limited to auristatin or a derivative and an analogue thereof, for example, monomethyl auristatin F (MMAF) and monomethyl auristatin E (MMAE), maytansinoid or a derivative and an analogue thereof, paclitaxel or a derivative and an analogue thereof, vinca-alkaloid or a derivative and an analogue thereof, cryptophycin or a derivative and an analogue thereof, and tubulysin or a derivative and an analogue thereof. The DNA damaging agent includes, but is not limited to one of doxorubicin or a derivative and an analogue thereof, calicheamicin or a derivative and an analogue thereof, camptothecin or a derivative and an analogue thereof, pyrrolobenzodiazepines (PBD) or a derivative and an analogue thereof, and duocarmycin or a derivative and an analogue thereof.

In this example, the MMAF is chosen as the drug, and TAMRA is chosen as the fluorescent molecule. The MMAF is subjected to a Michael addition reaction with the engineered cysteine on the antibody through a 6-maleimidocaproyl (mc) small-molecule linker, and the TAMRA is subjected to a Michael addition reaction with the engineered cysteine on the antibody through a PEG3-maleimide small-molecule linker.

### 1) Preparation of recombinant-fusion-nanobody-drug conjugates

The non-fused nanobody VHH0 and fused VHH1-VHH26 were respectively conjugated with mc-MMAF to obtain VHH0-MMAF and the recombinant-fusion-nanobody-drug conjugates, fused VHH1-MMAF to fused VHH26-MMAF. The preparation process was performed using the following method:
Firstly, 5 mM TCEP (Thermo Fisher Scientific) was added to reduce a sulfhydryl group of VHH0 and fused VHH1-VHH26 respectively for about 30 min at room temperature. Then, TCEP was removed by buffer-exchanging with PBS containing 1 mM EDTA followed by the addition of 2 eq of mc-MMAF to the protein solution for ligation at 4°C for 2 h. Finally, the solution was buffer-exchanged to remove the excess small molecules to afford the following conjugates: VHH0-MMAF, the recombinant-fusion-nanobody-drug conjugates, including fused VHH1-MMAF to fused VHH26-MMAF. The obtained recombinant-fusion-antibody-drug conjugates were characterized by SDS-PAGE (FIG. 4) and their molecular weights were determined by MALDI-TOF MS.

### 2) Preparation of recombinant-fusion-full-length-antibody-drug conjugates

The non-fused full-length antibody mAbO and fused mAb1-mAb3 were respectively conjugated with mc-MMAF to obtain mAb0-MMAF and recombinant-fusion-full-length-antibody-drug conjugates, fused mAb1-MMAF, fused mAb2-MMAF, and fused mAb3-MMAF. The preparation process was performed using the following method:

Firstly, antibodies and 100 mM of DTT were incubated for 16 h at 20°C, followed by buffer-exchanging with Tris-buffered saline (50 mM Tris-HCl at pH 7.5; 150 mM NaCl) to remove DTT. Next, 100 mM DHAA (> 15 eq) was added into the protein solution and the resulting mixture was incubated at room temperature for 3 h. In the reduction-reoxidation step, the natural interchain disulfide bonds were left intact (i.e., reoxidized) and only the engineered cysteines were reduced (i.e., unpaired). Finally, 4 eq of mc-MMAF was added for ligation at 4°C for 2 h, and the excess small molecules were removed by buffer-exchanging to obtain the following conjugates: mAb0-MMAF and the recombinant-fusion-full-length-antibody-drug conjugates, including fused mAb1-MMAF, fused mAb2-MMAF, and fused mAb3-MMAF. The obtained recombinant-fusion-full-length-antibody-drug conjugates were characterized by SDS-PAGE (FIG. 5) and their molecular weights were determined by LC-MS.

### Example 5

### In-vitro cytotoxicity

In this example, human breast cancer cell line SKBR3 with high expression of HER2 and human ovarian cancer cell line SKOV3 with high expression of HER2 were chosen to evaluate the ability of the recombinant-fusion-antibody-drug conjugates to inhibit the growth of cancer cells. The recombinant-fusion-antibody-drug conjugates of different concentrations were added to a 96-well plate seeded with SKBR3 or SKOV3 cells. After treatment for 48 h, the viability of cells was calculated using a CCK8 kit.

### 1) Cytotoxicity of the recombinant-fusion-nanobody-drug conjugates

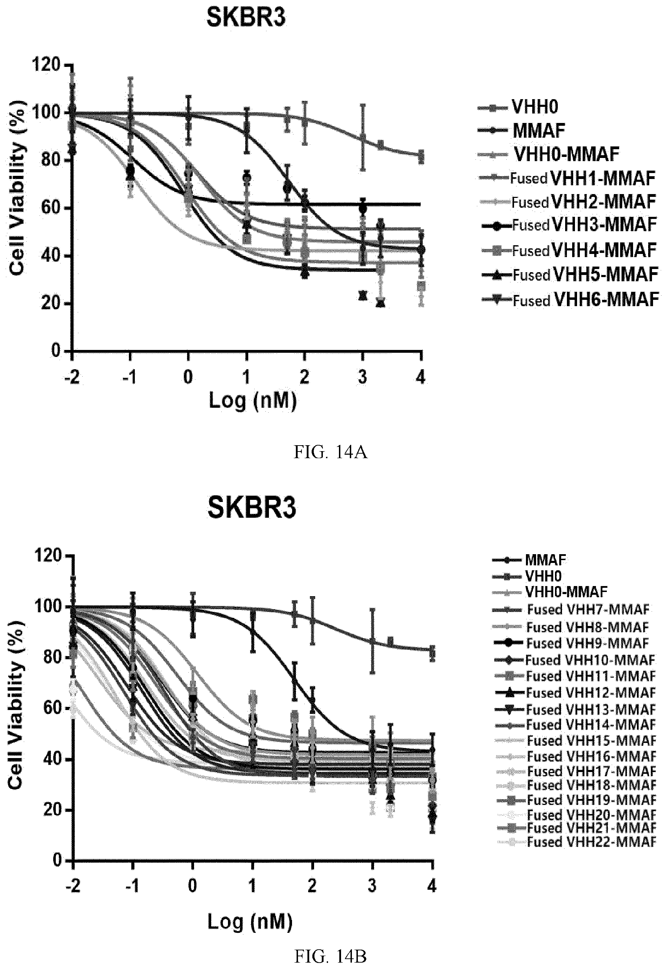

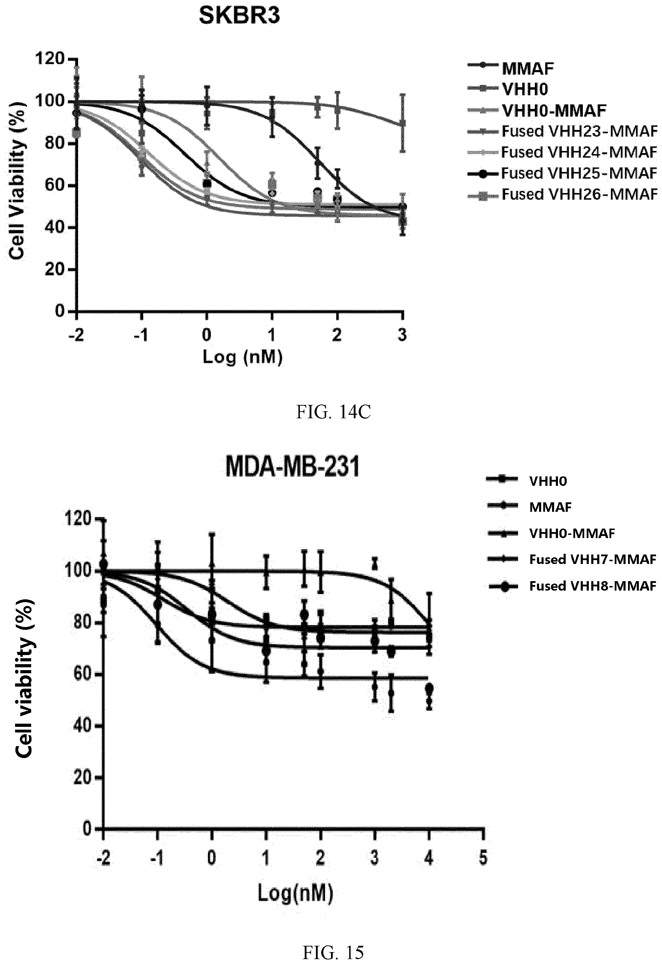

Cell viability was measured using different concentrations of MMAF, VHH0-MMAF, or different recombinant-fusion-nanobody-drug conjugates (fused VHH1-MMAF to fused VHH26-MMAF). The half-maximal inhibitory concentration IC₅₀ was calculated (FIGS. 14A-14C, and Table 7). FIGs. 14A, 14B and 14C respectively show the viability of SKBR3 cells treated with the recombinant-fusion-nanobody-drug conjugates, including fused VHH1-MMAF to fused VHH6-MMAF, fused VHH7-MMAF to fused VHH22-MMAF, and fused VHH23-MMAF to fused VHH26-MMAF. Table 7 shows the IC₅₀ values of MMAF, VHHO, VHH0-MMAF, and fused VHH1-MMAF to fused VHH26-MMAF. Data are expressed as the mean (n = 3).

| **Table 7 Half-maximal inhibitory concentration** | |
|---|---|
| **Name** | **IC₅₀ (nM)** |
| MMAF | 53.16 |
| VHH0 | n.a. |
| VHH0-MMAF | 1.573 |
| Fused VHH1-MMAF | 1.344 |
| Fused VHH2-MMAF | 0.126 |
| Fused VHH3-MMAF | 0.109 |
| Fused VHH4-MMAF | 0.885 |
| Fused VHH5-MMAF | 0.791 |
| Fused VHH6-MMAF | 0.791 |
| Fused VHH7-MMAF | 0.336 |
| Fused VHH8-MMAF | 0.3185 |
| Fused VHH9-MMAF | 0.294 |
| Fused VHH10-MMAF | 0.232 |
| Fused VHH11-MMAF | 0.160 |
| Fused VHH12-MMAF | 0.140 |
| Fused VHH13-MMAF | 0.0798 |
| Fused VHH14-MMAF | 0.069 |
| Fused VHH15-MMAF | 0.051 |
| Fused VHH16-MMAF | 0.027 |
| Fused VHH17-MMAF | 0.165 |
| Fused VHH18-MMAF | 0.394 |
| Fused VHH19-MMAF | 0.564 |
| Fused VHH20-MMAF | 0.038 |
| Fused VHH21-MMAF | 0.016 |
| Fused VHH22-MMAF | 0.014 |
| Fused VHH23-MMAF | 0.098 |
| Fused VHH24-MMAF | 0.128 |
| Fused VHH25-MMAF | 0.466 |
| Fused VHH26-MMAF | 0.095 |

The results show the following: 1) MMAF is cytotoxic with a maximal killing about 50%. 2) VHH0 is almost non-cytotoxic; the viability of the treated cells maintained approximately 100%. 3) VHH0-MMAF exhibits a significant cell-killing effect with a maximal killing about 50%. 4) Notably,the recombinant-fusion-nanobody-drug conjugates, including fused VHH1-MMAF to fused VHH26-MMAF, show superior cell-killing potency than VHH0-MMAF. These results are associated with those from the study of antibody colocalization with lysosomes. The recombinant-fusion-nanobody-drug conjugates with fusion of the lysosome-targeting peptides, including fused VHH1-MMAF to fused VHH26-MMAF, have a significantly higher killing potency against tumor cells than the nanobody-drug conjugate VHH0-MMAF, which is not fused with any lysosome-targeting peptide. In summary, the improved cytotoxicity of the recombinant-fusion-nanobody-drug conjugates is associated with their increased internalization and lysosome-targeting abilities.

In addition, in this example, the HER2-negative MDA-MB-231 cells were chosen as a negative control to compare the ability of fused VHH7-MMAF, fused VHH8-MMAF, VHH0-MMAF, VHHO, and MMAF to inhibit the growth of cancer cells. From FIG. 15, in HER2-negative MDA-MB-231 cells, only MMAF can trigger significant cell death; and none of the substances are cytotoxic, including fused VHH7-MMAF, fused VHH8-MMAF, VHH0-MMAF, and the VHH0. The result indicates that the recombinant-fusion-antibody-drug conjugates of the present disclosure exhibit a HER2-specific cell-killing effect, and thus the recombinant-fusion-antibody-drug conjugate can effectively target cancer cells.

### 2) Cytotoxicity of recombinant-fusion-full-length-antibody-drug conjugate

SKBR3 and SKOV3 cells were chosen to evaluate the ability of the recombinant-fusion-full-length-antibody-drug conjugates to inhibit the growth of cancer cells. Cell viability was measured using different concentrations of MMAF, mAb0-MMAF, or different recombinant-fusion-full-length-antibody-drug conjugates, including fused mAb1-MMAF to fused mAb3-MMAF. The half-maximal inhibitory concentration IC₅₀ was calculated.

### SKBR3

| **Table 8 Half-maximal inhibitory concentration** | |
|---|---|
| **Name** | **IC₅₀(nM)** |
| MMAF | 37.37 |
| mAbO | 0.432 |
| mAb-MMAF | 0.080 |
| Fused mAb1-MMAF | 0.034 |
| Fused mAb2-MMAF | 0.036 |
| Fused mAb3-MMAF | 0.034 |

### SKOV3

| **Table 9 Half-maximal inhibitory concentration** | |
|---|---|
| **Name** | **IC₅₀(nM)** |
| MMAF | 37.37 |
| mAb0 | 0.299 |
| mAb0-MMAF | 0.060 |
| Fused mAb1-MMAF | 0.020 |
| Fused mAb2-MMAF | 0.027 |
| Fused mAb3-MMAF | 0.022 |

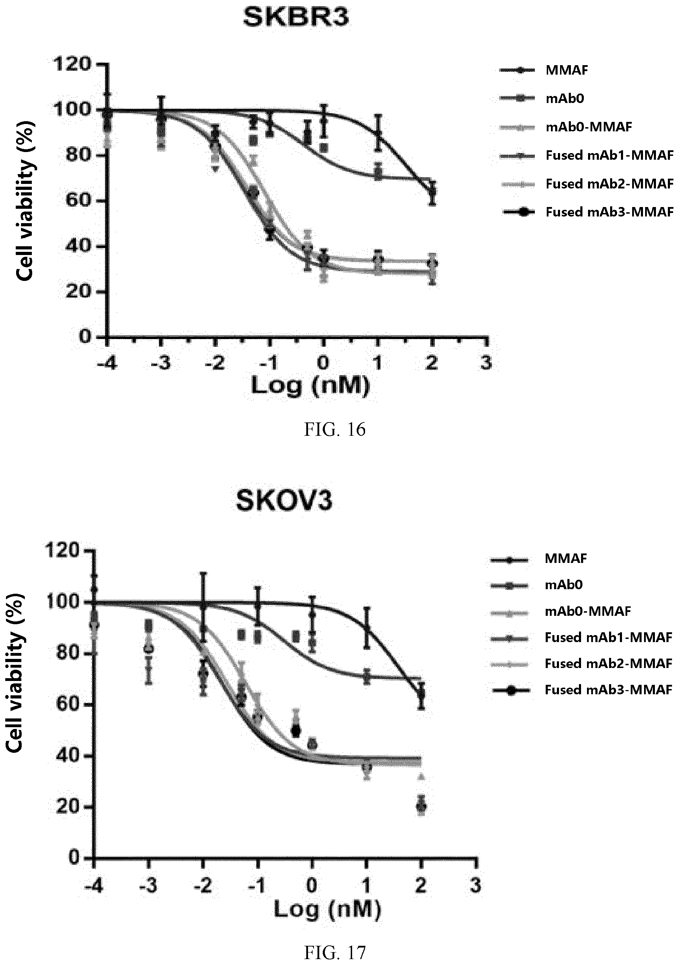

The cytotoxicity of the recombinant-fusion-antibody-drug conjugates in SKBR3 cells is shown in FIG. 16 and Table 8. FIG. 16 shows the viability of SKBR3 cells treated with the recombinant-fusion-full-length-antibody-drug conjugates. Table 8 shows the IC₅₀ values of MMAF, mAbO, mAb0-MMAF, fused mAb1-MMAF, fused mAb2-MMAF, and fused mAb3-MMAF in SKBR3 cells. The data are expressed as the mean (n = 3). The result indicates that the recombinant-fusion-full-length-antibody-drug conjugates with fusion with the lysosome-targeting peptides, including fused mAb1-MMAF, fused mAb2-MMAF, fused mAb3-MMAF, possess an excellent cancer-cell-killing potency, with IC₅₀ values in the picomolar range, having a significant improvement compared to the non-fused full-length-antibody-drug conjugate mAbO-MMAF.

The cytotoxicity of the recombinant-fusion-antibody-drug conjugates in SKOV3 cells is shown in FIG. 17 and Table 9. FIG. 17 shows the viability of SKOV3 cells treated with the recombinant-fusion-full-length-antibody-drug conjugates. Table 9 shows the IC₅₀ values of MMAF, mAbO, mAbO-MMAF, fused mAb1-MMAF, fused mAb2-MMAF, and fused mAb3-MMAF in SKOV3 cells. Data are expressed as the mean (n = 3). The result confirms that the recombinant-fusion-full-length-antibody-drug conjugates with fusion of the lysosome-targeting peptides, including fused mAb1-MMAF to the fused mAb3-MMAF, have a higher cancer-cell-killing potency than the non-fused full-length-antibody-drug conjugate mAb0-MMAF.

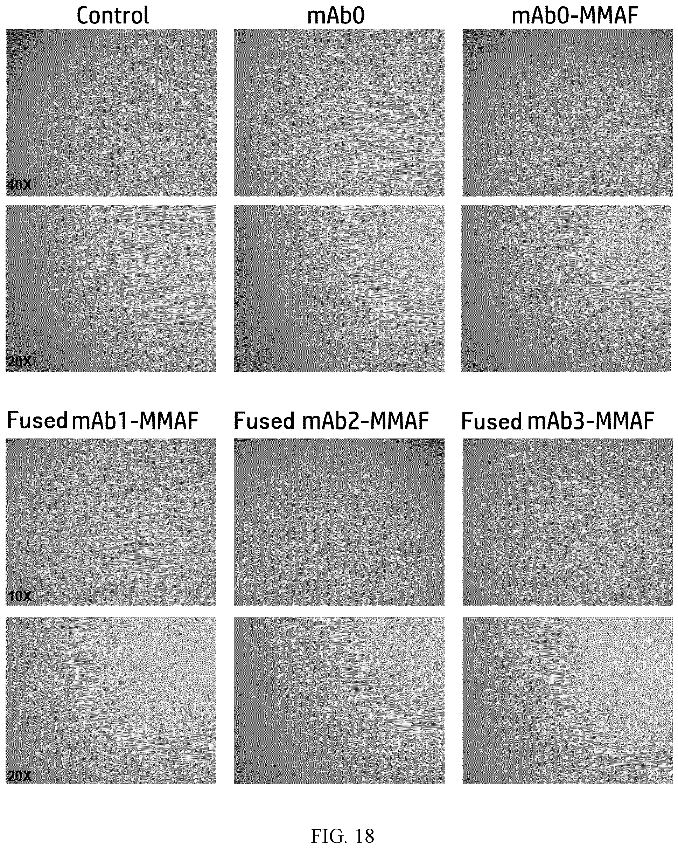

Finally, SKOV3 cells with different treatments were visualized through microscopic imaging. The procedure was as follows: SKOV3 cells were plated in a 96-well plate (~8,000 cells per well) and incubated in monolayer overnight. Cells were then divided into six groups (blank control, mAbO, mAb0-MMAF, fused mAb1-MMAF, fused mAb2-MMAF, and fused mAb3-MMAF). Except for the blank control group, the other five groups were treated the corresponding drugs at 0.01 nM for 48 h; the medium was aspirated off and cells were fixed with 4% paraformaldehyde for 15 min at room temperature. Lastly, the 4% paraformaldehyde solution was aspirated off followed by the addition of 100 µL PBS. Cell were visualized with a microscope. The images of SKOV3 cells with different treatments are shown in FIG. 18.

From FIG. 18, cells were in a healthy state in the blank control group, in which cells were under no treatment; and cell did not show any significant change in the mAbO group. Compared to the blank control group and the mAbO group, cells changed significantly in the mAb0-MMAF group, the fused mAb1-MMAF group, the fused mAb2-MMAF group, and the fused mAb3-MMAF group. The morphology of the cells treated with the recombinant-fusion-full-length-antibody-drug conjugates became irregular, indicating that fused mAb1-MMAF/ fused mAb2-MMAF/ fused mAb3-MMAF can substantially affect the viability of SKOV3 cells and have a higher cytotoxicity compared to mAb0-MMAF.

In conclusion, the recombinan-fusion-full-length-antibody-drug conjugates with fusion of the lysosome-targeting peptides have a significant cancer-cell-killing potency, with IC₅₀ values in the picomolar range. The recombinant fusion of the antibodies with the lysosome-targeting peptides can effectively improve the cytotoxicity of the corresponding full-length-antibody-drug conjugates. The cytotoxicity of the recombinant-fusion-antibody-drug conjugates is associated with their internalization and lysosome-targeting abilities.

### Example 6

### Anti-tumor activity in vivo

In this example, the anti-tumor activity of the recombinant-fusion-antibody-drug conjugates was evaluated in xenograft models using human breast cancer cell line BT474 and human gastric cancer cell line NCI-N87, respectively.

Female nude mice (6-8 weeks old) were divided into the following 3 groups: normal saline group, mAb0-MMAF group, and fused mAb3-MMAF group. The BT474 or NCI-N87 cells (5×10⁶-7×10⁶) were subcutaneously inoculated to the right-front flank of the nude mice. Post-transplantation, the length and width of tumor were measured by a Vernier caliper. When tumors reached 100-300 mm³, according to the grouping, mice were administered via tail vein injections with a single drug dose of 10 mg/kg (100 µL). Mice bearing BT474 tumors were administrated every 5 days and 6 times in total. Mice bearing NCI-N87 tumors were administrated every 5 days and 8 times in total. The body weight and tumor size of nude mice were periodically measured; the length and the width were respectively represented by L and W, and the tumor volume was calculated using the following formula: V = [length (mm)×width² (mm)²]/2. Mice were weighed again at 24 hours after the last drug administration and sacrificed by cervical vertebra dislocation.

### 1) Anti-tumor activity of recombinant-fusion-antibody-drug conjugate in a BT474 xenograft mouse model

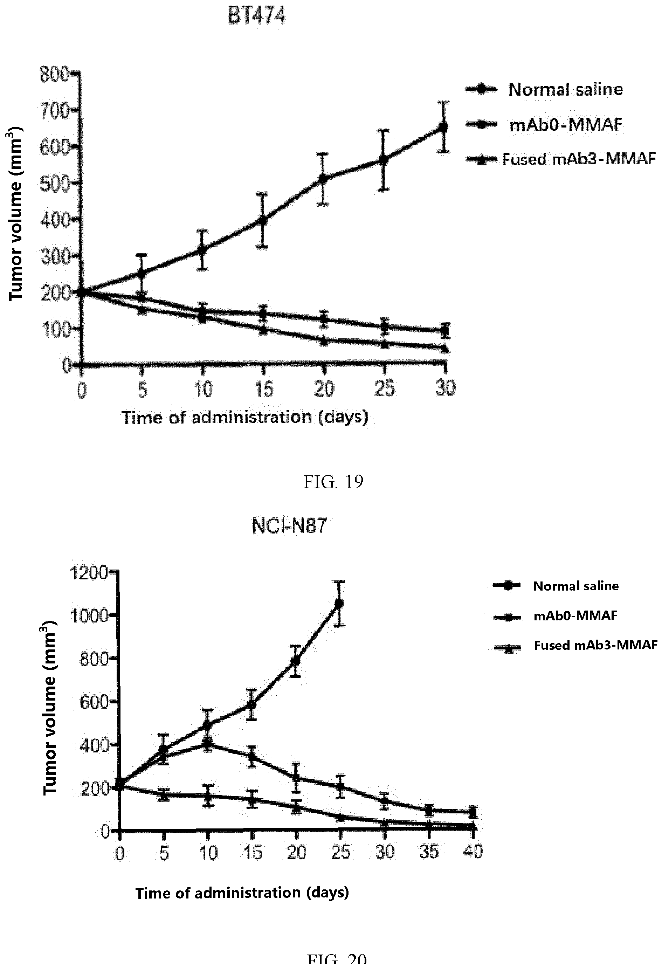

FIG. 19 shows the anti-tumor activity of the recombinant-fusion-antibody-drug conjugates in mice bearing BT474 tumors, wherein the X-axis represents the number of days of administration and the Y-axis represents the tumor volume. The result indicates that both mAb0-MMAF and fused mAb3-MMAF inhibite tumor growth compared to the control group. However, fused mAb3-MMAF exhibits a superior effect in suppressing the growth of tumors. Therefore, it can be proved that the recombinant-fusion-antibody-drug conjugates of the present disclosure have improved anti-tumor effects against human breast cancers.

In addition, the administration of mAbO-MMAF and fused mAb3-MMAF did not cause weight loss in mice, suggesting that the recombinant-fusion-antibody-drug conjugates of the present disclosure have a favorable safety profile.

### 2) Anti-tumor activity of recombinant-fusion-antibody-drug conjugate in an NCI-N87 xenograft mouse model.

FIG. 20 shows the anti-tumor activity of the recombinant-fusion-antibody-drug conjugates in mice bearing NCI-N87 tumors, wherein the X-axis represents the number of days of administration and the Y-axis represents the tumor volume. The result shows that both mAb0-MMAF and fused mAb3-MMAF suppress tumor growth compared to the control group. However, fused mAb3-MMAF exhibits a superior effect on suppressing the growth of tumors. Therefore, it can be proved that the recombinant-fusion-antibody-drug conjugates of the present disclosure have improved anti-tumor effect against human gastric cancers.

In addition, the administration of mAb0-MMAF and mAb3-MMAF did not cause weight loss in the mice, suggesting that the recombinant-fusion-antibody-drug conjugates of the present disclosure could have a favorable safety profile.

Although the present disclosure is disclosed as the foregoing in the manner of embodiments, however, which are not used to limit the present disclosure, any person skilled in the art can make various changes and modification without departing from the spirits and scope of the present disclosure. Therefore, the protection scope of the present disclosure is determined by the attached claims.

## Claims

1. A recombinant fusion antibody, comprising an antibody, at least one lysosome-targeting peptide, and at least one linker peptide, wherein the antibody is fused with the lysosome-targeting peptide via the linker peptide to form the recombinant fusion antibody;
wherein the lysosome-targeting peptide is at least one selected from the group consisting of a lysosome-sorting signal peptide from a Golgi-localized, γ-adaptin ear-containing, ARF-binding protein (GGA), a lysosome-sorting signal peptide from a mannose 6-phosphate receptor (MPR), a lysosome-sorting signal peptide from a lysosome-associated membrane protein (LAMP), and and a variant; and
wherein the variant is a peptide with one or more amino acids substituted, deleted, and/or added when aligned with the lysosome-sorting signal peptide from a GGA, a MPR or a LAMP.

2. The recombinant fusion antibody according to claim 1, wherein the lysosome-targeting peptide is at least one selected from the group consisting of the following amino acid sequences: ASVSLLDDELMSL (SEQ ID NO: 1), RRRASVSLLDDELMSL (SEQ ID NO: 2), ASVSLLDDEL (SEQ ID NO: 3), ASSGLDDLDLLGK (SEQ ID NO: 4), VQNPSADRNLLDL (SEQ ID NO: 5), NALSWLDEELLCL (SEQ ID NO: 6), SDEDLLHI (SEQ ID NO: 7), SFHDDSDEDLLHI (SEQ ID NO: 8), EESEERDDHLLPM (SEQ ID NO: 9), SYKYSKVNKE (SEQ ID NO: 10), PAAYRGVGDD (SEQ ID NO: 11), RRRSDEDLLHI (SEQ ID NO: 12), RRLRKSDEDLLHI (SEQ ID NO: 13), RRRRKSDEDLLHI (SEQ ID NO: 14), RRRSFHDDSDEDLLHI (SEQ ID NO: 15), RRLRKSFHDDSDEDLLHI (SEQ ID NO: 16), RRRRKSFHDDSDEDLLHI (SEQ ID NO: 17), RKRSHAGYQTI (SEQ ID NO: 18), RRRRKRKRSHAGYQTI (SEQ ID NO: 19), KHHHAGYEQF (SEQ ID NO: 20), and RRLRKHHHAGYEQF (SEQ ID NO: 21).

3. The recombinant fusion antibody according to claim 1, wherein the amino acid sequence of the linker peptide is (Leu-Pro-Glu-Thr)ₓ-(Gly_{y1}-Ser_{y2})_{z}, wherein x = 0 or 1, y₁ = 3, 4 or 5, yz = 0 or 1, and z = 1, 2 or 3.

4. The recombinant fusion antibody according to claim 3, wherein the linker peptide is at least one selected from the group consisting of the following amino acid sequences: Leu-Pro-Glu-Thr-Gly-Gly-Gly (SEQ ID NO: 22), Gly-Gly-Gly, Gly-Gly-Gly-Gly-Gly (SEQ ID NO: 23), Gly-Gly-Gly-Gly-Ser (SEQ ID NO: 24), Gly-Gly-Gly-Gly-Ser-Gly-Gly-Gly-Gly-Ser (SEQ ID NO: 25), and Gly-Gly-Gly-Gly-Ser-Gly-Gly-Gly-Gly-Ser-Gly-Gly-Gly-Gly-Ser (SEQ ID NO: 26).

5. The recombinant fusion antibody according to claim 1, wherein the antibody is capable of binding to a target protein on the surface of a tumor cell, and the target protein on the surface of the tumor cell is at least one selected from the group consisting of the following: EGFR, EGFRvIII, HER2, ErB3, CD19, CD20, CD30, CD33, CD37, CD46, CD48, CD74, CD79B, CD27 ligand, TROP2, Nectin-4, PSMA, BCMA, HGFR, FOLR1, CA125, ALCAM, SLC1A5, Siglec-2, Siglec-3, IL-2R alpha, IL-3R alpha, TNF-alpha, B7-H3, GUCY2C, TPBG, ROR2, ENPP3, Coagulation Factor III, Mesothelin, Transferrin R, CEACAM-5, IGF-IR, Syndecan-1, DLL3, EpCAM, LIV-1, ROR1, TIM-1, Ly6E, and NCAM-1.

6. The recombinant fusion antibody according to claim 1, wherein the antibody is a monospecific antibody, a bispecific antibody, or a multispecific antibody.

7. The recombinant fusion antibody according to claim 1, wherein the antibody is a chimeric antibody, a humanized antibody, or a human antibody.

8. The recombinant fusion antibody according to claim 1, wherein the antibody comprises a monoclonal antibody or an antigen-binding fragment thereof.

9. The recombinant fusion antibody according to claim 8, wherein the antigen-binding fragment is Fab, Fab', F(ab')₂, Fv, dsFv, scFv, sc(Fv)₂, or VHH.

10. The recombinant fusion antibody according to claim 8, wherein a fusion site of the lysosome-targeting peptide comprises at least one selected from the group consisting of a C-terminus of a heavy chain of the monoclonal antibody, an N-terminus of the heavy chain of the monoclonal antibody, a C-terminus of a light chain of the monoclonal antibody, and an N-terminus of the light chain of the monoclonal antibody.

11. The recombinant fusion antibody according to claim 8, wherein a fusion site of the lysosome-targeting peptide comprises at least one selected from the group consisting of a C-terminus of the antigen-binding fragment and an N-terminus of the antigen-binding fragment.

12. A recombinant-fusion-antibody-drug conjugate, having the structure as follows:
Drₙ₁Ab,
wherein Dr is a drug;
Ab is the recombinant fusion antibody according to any one of claims 1-11; and
n1 is an integer greater than or equal to 1.

13. The recombinant-fusion-antibody-drug conjugate according to claim 12, wherein the drug is a cytotoxic agent selected from any one of the following:
a microtubule inhibitor or a prodrug thereof,
a DNA damaging agent or a prodrug thereof,
an RNA polymerase II inhibitor, and
a toxin from the bacterial, fungal or animal origin.

14. The recombinant-fusion-antibody-drug conjugate according to claim 13, wherein the microtubule inhibitor is selected from any one of the following: auristatin or a derivative and an analogue thereof, maytansinoid or a derivative and an analogue thereof, paclitaxel or a derivative and an analogue thereof, vinca-alkaloid or a derivative and an analogue thereof, cryptophycin or a derivative and an analogue thereof, and tubulysin or a derivative and an analogue thereof.

15. The recombinant-fusion-antibody-drug conjugate according to claim 13, wherein the DNA damaging agent is selected from any one of the following: doxorubicin or a derivative and an analogue thereof, calicheamicin or a derivative and an analogue thereof, camptothecin or a derivative and an analogue thereof, pyrrolobenzodiazepine (PBD) or a derivative and an analogue thereof, and duocarmycin or a derivative and an analogue thereof.

16. The recombinant-fusion-antibody-drug conjugate according to claim 12, wherein the drug is conjugated to a reactive group on the recombinant fusion antibody through a covalent bond.

17. The recombinant-fusion-antibody-drug conjugate according to claim 16, wherein the reactive group on the recombinant fusion antibody is a lysine, a cysteine or a bioorthogonal group.

18. The recombinant-fusion-antibody-drug conjugate according to claim 17, wherein the cysteine is a native cysteine from the recombinant fusion antibody or an engineered cysteine obtained by artificial mutation.

19. The recombinant-fusion-antibody-drug conjugate according to claim 17, wherein the bioorthogonal group is one of an azide group, an alkynyl group, an aldehyde group, a ketone group, and a fluorosulfate group.

20. The recombinant-fusion-antibody-drug conjugate according to claim 12, wherein the recombinant fusion antibody is conjugated with the drug via a cleavable or non-cleavable small-molecule linker.

21. The recombinant-fusion-antibody-drug conjugate according to claim 20, wherein the small-molecule linker is selected from any one of the following: a peptide, an oligosaccharide, - (CH₂)ₙ-, -(CH₂CH₂O)ₙ-, Val-Cit-PABC, Val-Ala-PABC, Val-Lys(Ac)-PABC, Phe-Lys-PABC, Phe-Lys(Ac)-PABC, D-Val-Leu-Lys, Gly-Gly-Arg, Ala-Ala-Asn-PABC, Ala-PABC, and PABC or a combination thereof, wherein n is an integer of 1-10, Cit is citrulline, Ac is acetyl, and PABC is p-aminobenzyl alcohol.

22. Use of a recombinant-fusion-antibody-drug conjugate according to any one of claims 12-21 in the preparation of a medicament for treating cancer.

23. The use according to claim 22, wherein the cancer is a gastric cancer, an intestinal cancer, a liver cancer, a lung cancer, a pancreatic cancer, a breast cancer, a cervical cancer, an endometrial cancer, an ovarian cancer, a prostate cancer, a bladder cancer, a nasopharyngeal/throat or soft tissue cancer, a blood cancer or lymphoma, and a skin cancer.
